(19) Europäisches Patentamt
European Patent Office
Office européen des brevets

(11) **EP 2 200 675 B1**

(12) **EUROPÄISCHE PATENTSCHRIFT**

(45) Veröffentlichungstag und Bekanntmachung des Hinweises auf die Patenterteilung:
**10.02.2016  Patentblatt 2016/06**

(21) Anmeldenummer: **08782833.1**

(22) Anmeldetag: **28.08.2008**

(51) Int Cl.:
*A61M 1/36* (2006.01)    *A61M 1/34* (2006.01)

(86) Internationale Anmeldenummer:
**PCT/AT2008/000305**

(87) Internationale Veröffentlichungsnummer:
**WO 2009/026603 (05.03.2009 Gazette 2009/10)**

(54) **VERFAHREN ZUM ERFASSEN DER IONENKONZENTRATION BEI CITRAT-ANTIKOAGULIERTER EXTRAKORPORALER BLUTREINIGUNG**

METHOD FOR DETECTING THE ION CONCENTRATIONS OF CITRATE ANTI-COAGULATED EXTRACORPOREAL BLOOD PURIFICATION

PROCÉDÉ SERVANT À DÉTERMINER LA CONCENTRATION IONIQUE DANS UNE OPÉRATION EXTRACORPORELLE DE PURIFICATION DE SANG SOUMIS À UNE ANTICOAGULATION AU CITRATE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **31.08.2007  AT 13682007**

(43) Veröffentlichungstag der Anmeldung:
**30.06.2010  Patentblatt 2010/26**

(73) Patentinhaber: **Zentrum für biomedizinische Technologie der Donau-Universität Krems
3500 Krems (AT)**

(72) Erfinder:
• **BRANDL, Martin
A-3620 Spitz (AT)**
• **HARTMANN, Jens
3511 Furth (AT)**
• **STROBL, Karin
3491 Strass (AT)**
• **FALKENHAGEN, Dieter
3500 Krems (AT)**

(74) Vertreter: **Patentanwaltskanzlei
Matschnig & Forsthuber OG
Biberstraße 22
Postfach 36
1010 Wien (AT)**

(56) Entgegenhaltungen:
**DE-A1- 10 114 283      DE-A1- 19 823 570
US-A1- 2007 066 928**

**Beschreibung**

Die Erfindung bezieht sich auf eine Vorrichtung zur Citrat-antikoagulierten extrakorporalen Blutreinigung, aufweisend: ein extrakorporales Blutreinigungssystem mit einem extrakorporalen Blutkreislauf, welcher eine Dialyseeinheit, einen Blutzulauf zur Dialyseeinheit für von einem Patienten entnommenes Blut und einen Blutablauf von der Dialyseeinheit für in den Patienten zurückzuführendes Blut aufweist, eine gesteuerte Citrat-Dosiereinrichtung zur Zuführung von Citrat an einer Citratzufuhrstelle stromauf der Dialyseeinheit, eine gesteuerte Substitutionsmedium-Dosiereinrichtung zur Zuführung eines Substitutionsmediums an einer Substitutionsmedium-Zufuhrstelle stromab der Dialyseeinheit, zumindest ein Ionenkonzentrationsmessmittel zur Messung zweiwertiger Kationen und eine Steuerung, welche mit dem zumindest einen Ionenkonzentrationsmessmittel sowie der Citrat-Dosiereinrichtung und der Substitutionsmedium-Dosiereinrichtung verbunden ist, wobei die Steuerung dazu eingerichtet ist, die Dosierung des Substitutionsmediums in Abhängigkeit eines Vergleichs zwischen einem Sollwertbereich und der mittels des Ionenkonzentrationsmessmittels gemessenen Ionenkonzentration zu regeln.

Weiters bezieht sich die Erfindung auf ein Verfahren zum Erfassen einer Ionenkonzentration zweiwertiger Kationen des Blutes während einer Citrat-antikoagulierten extrakorporalen Blutreinigung, bei welchem an einem extrakorporalen Blutkreislauf an einer stromauf einer Dialyseeinheit befindlichen Citratzufuhrstelle Citrat und an einer stromab der Dialyseeinheit befindlichen Substitutionsmedium-Zufuhrstelle ein Substitutionsmedium in den extrakorporalen Blutkreislauf eingebracht werden und die Steuerung der Dosierung des Substitutionsmediums in Abhängigkeit eines Vergleichs zwischen einem Sollwertbereich und der gemessenen Ionenkonzentration erfolgt.

Bei den meisten Patienten, die einer extrakorporalen Blutreinigung bedürfen, ist eine Hemmung der Blutgerinnung erforderlich. Der Kontakt von Blut bzw. Plasma mit Fremdoberflächen führt zur Kontaktaktivierung und in weiterer Folge zu Gerinnung, Freisetzung von Kininen und zur Aktivierung des Komplementsystems. Diese Prozesse werden auch durch extrakorporale Blutreinigungsverfahren ausgelöst, da Plasma bzw. Blut im Rahmen dieser Verfahren mit Membranen und eventuell Adsorbermaterialien in Kontakt gebracht wird. Aufgrund der hohen Anforderungen an die Patientensicherheit und der hohen Prävalenz für blutreinigende Therapien, ist eine sichere, effektive und leicht handzuhabende Antikoagulation essenziell.

Als derzeitiger Standard wird in der klinischen Behandlung von Hämodialysepatienten Heparin eingesetzt, welches durch Infusion in den arteriellen Schenkel des extrakorporalen Blutkreislaufes eingebracht wird. Heparin kann jedoch zu Komplikationen führen, wie z.B. HIT (Heparin Induced Thrombocytopenia)_[Hetzel GR, Sucker C (2005), Nephrol Dial Transplant, 20 (10): 2036-2042; M. Franchini (2005), Thrombosis Journal, 3 (14)], Heparin-Bindung an den Adsorber oder Filter oder eine unerwünschte intrakorporale Antikoagulation, wobei letztere besonders bei Patienten mit erhöhtem Blutungsrisiko problematisch werden kann.

Eine Alternative zur Heparinmethode stellt die Antikoagulation mit Citrat dar [Janssen MJ et al. (1993), Nephrol Dial Transplant, 8 (11): 1228-1233; Pinnick et al. (1983), N Engl J Med, Vol. 308, 258-261]. Die antikoagulatorische Wirkung des Citrats beruht auf dessen Komplexbildung mit freien zweiwertigen Kationen, wie Kalziumionen und Magnesiumionen. Ionisiertes Kalzium ist ein wichtiger Cofaktor im Gerinnungssystem, da es eine katalytische Wirkung auf die meisten Enzyme der Gerinnungskaskade hat. Eine Komplexierung der Kalziumionen durch Citrat führt folglich zu einer Unterbindung des Ablaufs der Gerinnungskaskade. Eingesetzte Konzentrationen reichen von 2 bis 7.4 mmol Citrat pro Liter Blut [Palsson R, Niles JL (1999), Kidney Int 55: 1991-1997; Böhler et al. (1996), J Am Soc of Nephrol. 7(2), 234-241], wobei Konzentrationen des ionisierten Kalziums von 0,25 mmol/l bis 0,35 mmol/l [Kutsogiannis et al. (2000), Am J Kidney Dis, Vol. 35, 802-811; Tolwani et al. (2001), Kidney International, Vol. 60, 370-374] bzw. < 0,4 mmol/l [Swartz et al. (2004), Clinical Nephrology, Vol. 61(2),134-143] als Zielwerte angegeben werden.

Ein extrakorporaler Blutkreislauf setzt sich im Wesentlichen aus einer Filtereinheit, einem Blutzulauf vom Patienten zur Filtereinheit (arterieller Schenkel) und einem Blutablauf von der Filtereinheit zum Patienten (venöser Schenkel) zusammen. Das Blut wird mittels einer Blutpumpe durch den Kreislauf gepumpt. Der Blutfluss [ml/min] ist, wie weiter unten beschrieben, ein wichtiger Parameter für das System und stellt das Blutvolumen, das pro Zeiteinheit von der Blutpumpe gepumpt wird, dar. Die Filtereinheit beinhaltet üblicherweise ein Dialysefilter, ein Plasmafilter bzw. ein Hämofilter oder eine Kombination davon. Die Membran-Blutreinigungsverfahren beruhen auf den physikalischen Vorgängen der Diffusion und Konvektion. Im Dialysefilter befindet sich eine semipermeable Membran, die das Blut von der Dialyseflüssigkeit trennt. Dabei diffundieren niedermolekulare Substanzen aus dem Blut in die Dialyseflüssigkeit und werden dadurch aus dem Blut entfernt. Das Ausmaß der Entfernung der Substanzen wird als Clearance bezeichnet und ist vom Dialysefilter sowie Blut- und Dialysatfluss abhängig. Bei der Dialyse kann dem Patienten mittels Transmembrandruck auch im Körper angesammelte Flüssigkeit entzogen werden. Dieser Vorgang wird auch als Ultrafiltration bezeichnet. Bei der Hämofiltration wird Filtrat an einer Ultrafiltrationsmembran abgepresst und durch Substitutionslösung ersetzt. Das Filtrat enthält - je nach eingesetztem Filter - niedermolekulare oder nieder- und hochmolekulare Substanzen. Bei einem weiteren bekannten Blutreinigungsverfahren wird Filtrat über ein Hämo- oder Plasmafilter abgepresst und in einem Plasmakreislauf rezirkuliert, gereinigt (z.B. mittels Adsorber oder mittels MDS - Microspheres Detoxification System [EP 0776223, US 5855782] und abschließend in den extrakorporalen Blutkreislauf rückgeführt. Neben den Membran-Blutreinigungssystemen gibt es Adsorptionssysteme, bei welchem das Blut oder das Plasma einen Adsorber passiert und durch physikochemische Prozesse gereinigt wird. Üblicherweise besteht ein Adsorber aus einer aktiven Matrix, die unerwünschte Blutbestandteile spezifisch aus dem Blut entfernt. Selbstverständlich ist die Citrat-Antikoagulation für Adsorbersysteme ebenfalls geeignet, allerdings wird ein Dialysefilter vorteilhafterweise stromab dem Adsorberkreislauf angeordnet, um - wie weiter unten beschrieben - das Citrat vor der Re-Infusion des Blutes teilweise aus dem Blutkreislauf zu entfernen.

Die Citratzufuhr erfolgt mittels einer Infusion in den arteriellen Schenkel des extrakorporalen Blutkreislaufes. Das derart in seiner Gerinnung gehemmte Blut wird in Membran- bzw. Membran/Adsorption- basierten Systemen gereinigt. Das Citrat sowie der Citrat-Kalzium-Komplex werden durch die Dialyse in Abhängigkeit von dem verwendeten Dialysefilter zum größten Teil aus dem extrakorporalen Blutkreislauf wieder entfernt, sodass nur ein geringer Teil des infundierten Citrats in den Blutkreislauf des Patienten gelangt. Für die Citrat-Antikoagulation ist somit eine Dialyseeinheit vorteilhaft. Citrat wird im menschlichen Körper zu $CO_2$ und Wasser metabolisiert, wobei die Citrat-Belastung des Patienten die metabolische Rate nicht wesentlich übersteigen darf, um eine pathophysiologisch relevante Auswirkung auf den Säuren/ Basen-Haushalt zu vermeiden. Um das Gerinnungssystem und sonstige physiologische Funktionen von Kalzium- und Magnesiumionen im Patienten nicht zu verändern, ist eine Substitution dieser Ionen notwendig. Dabei wird ein kalziumhältiges oder ein kalzium- und magnesiumhältiges Substitutionsmedium entweder durch Infusion in den venösen Schenkel des extrakorporalen Blutkreislaufes oder über einen separaten Venenzugang in das Blut eingebracht.

Der wichtigste therapeutische Vorteil der Citrat-Antikoagulation liegt darin, dass - im Gegensatz zur Heparin-Antikoagulation - die Blutgerinnung ausschließlich im extrakorporalen Kreislauf gehemmt wird und unerwünschte intrakorporale Blutungen dadurch vermieden werden. Neben der Antikoagulation verbessert ein Citratzusatz wesentlich die Biokompatibilität eines extrakorporalen Blutreinigungssystems, da die Komplementaktivierung, die der Anwesenheit freier Kalzium- und Magnesiumionen bedarf, durch Komplexierung dieser Ionen unterdrückt wird. Weiters kann die Citrat-Antikoagulation bei Patienten, bei welchen Heparin-Antikoagulation kontraindiziert ist (HIT) eingesetzt werden. Durch verlängerte Filterstandzeiten beim Einsatz von Citrat-Antikoagulation ist diese besonders für Langzeitbehandlungen wie z.B. in der Akutdialyse geeignet.

Obwohl die Vorteile der Citrat-Antikoagulation gegenüber der Heparin-Standardmethode klar auf der Hand liegen, kommt sie in der klinischen Behandlung nur selten und nur in wenigen Blutreinigungseinrichtungen zur Anwendung. Warum sich die Methode trotz ihrer Vorteile für den Patienten noch nicht durchgesetzt hat, lässt sich durch folgende Gründe erklären: Einerseits ist die Citrat-Antikoagulation etwas aufwändiger als die Heparinmethode und andererseits ist sie durch die Komplexität der Dosierung und die fehlende Automatisierung und Standardisierung mit einem Sicherheitsrisiko verbunden, erfordert eine sorgfältige Überwachung und wird folglich ausschließlich von erfahrenen Experten durchgeführt. Bei Änderung des extrakorporalen Blutflusses müssen die Zufuhrraten für Citrat und für das Substitutionsmedium manuell angepasst werden. Eine falsche Dosierung, die beispielsweise durch einen Bedienungsfehler, eine fehlerhafte Einstellung der Zufuhrraten oder einen Infusionspumpenausfall verursacht wird, kann zu Komplikationen wie beispielsweise Hypokalzämie führen. Dies ist ein Zustand, der lebensbedrohliche Ausmaße annehmen kann. Die Dosierung sollte anhand der ermittelten Ionenkonzentration des Patientenblutes - vorteilhafterweise der Kalziumionenkonzentration - eingestellt werden. Nur durch eine möglichst genaue sowie sehr engmaschige oder kontinuierliche Messung der Kalziumionenkonzentration des Blutes, durch welche eine ebenso engmaschige oder kontinuierliche Anpassung der Dosierung der Infusionslösungen veranlasst wird, kann das Risiko eines unerwünschten unphysiologischen Zustands minimiert und die Sicherheit des Patienten gewährleistet werden. In Anbetracht der Notwendigkeit für ein Antikoagulationssystem, welches auch für Patienten geeignet ist, für die eine Anwendung der Heparinmethode nachteilig oder nicht möglich ist, und der fortschreitenden Überalterung der Bevölkerung und der damit verbundenen steigenden Prävalenz für blutreinigende Therapien, besteht daher großer Bedarf an einem verlässlichen und effektiven sowie automatisierten und standardisierten Citrat-Antikoagulationssystem, mit welchem die Patientensicherheit möglichst hoch gehalten werden kann.

In der WO 91/06326 wird ein Dialyseverfahren mit Citrat-Antikoagulation zur Anwendung in der Hämodialyse vorgestellt. Das Citrat wird in den arteriellen Schenkel des extrakorporalen Blutkreislaufs infundiert, wobei die Citratzufuhrrate in Abhängigkeit der Blutflussrate eingestellt wird. Kalziumionen werden über einen separaten venösen Zugang substituiert, wobei die Zufuhrrate der Ca-Ionen-Substitutionslösung anhand der Citratzufuhrrate und der in mehrstündigen Zeitintervallen ermittelten Ca-Ionenkonzentration entnommener Blutproben eingestellt wird. Eine engmaschige bzw. kontinuierliche Überwachung der Ca-Ionenkonzentration erfolgt bei dem in der WO 91/06326 beschriebenen Verfahren nicht, und es kann daher, wie oben bereits ausgeführt, die Sicherheit des Patienten nicht vollständig gewährleistet werden. Die fehlende Standardisierung und Automatisierung ist zusätzlich stark limitierend für die Patientensicherheit und eine benutzerfreundliche Anwendung des Systems.

Eine Vorrichtung sowie ein Verfahren für die Citrat-antikoagulierte extrakorporale Blutreinigung ist der US 2007/066928 A1 zu entnehmen. Das Dokument offenbart ein Mittel zur Erfassung einer Ionenkonzentration, welches unter anderem zweiwertige Kationen wie Calzium- und Magnesiumionen misst und stromab der Dialyseeinheit im extrakorporalen Blutkreislauf angeordnet ist. Die Dosierung Citrat-hältiger Lösungen, welche vor und nach der Dialyseeinheit in den extrakorporalen Blutkreislauf infundiert werden können, wird in Abhängigkeit der gemessenen Ionenkonzentration geregelt. Die Dosierung der Kalzium- und Magnesium-hältigen Elektrolytlösung erfolgt unabhängig von der gemessenen Ionenkonzentration. Nachteilig an der in der US 2007/066928 gezeigten Vorrichtung bzw. dem gezeigten Verfahren ist vor allem, dass es nicht möglich ist, auf den intrakorporalen physiologischen Zustand des Patienten rückzuschließen. Komplikationen wie beispielsweise eine Hypokalzämie in Folge einer Falschdosierung der Citrat- bzw. der Elektrolytlösung können nicht rechtzeitig erkannt werden. Die Patientensicherheit kann daher nicht vollständig gewährleistet werden.

Eine weitere Vorrichtung sowie ein Verfahren für die Citrat-antikoagulierte Blutreinigung ist in der US 2004/133145A1 gezeigt. Die Infusionsraten für das Citrat und für das Substitutionsmedium werden in Abhängigkeit gemessener Flussraten eingestellt. Auch bei dieser Vorrichtung bzw. diesem Verfahren kann die Patientensicherheit nicht vollständig gewährleistet werden.

In der US 2007/0007184 A1 ist ein extrakorporales Blutreinigungssystem, allerdings ohne Citrat-Antikoagulationssystem, offenbart. Das Blutreinigungssystem weist einen Einweg-Sensor auf, mit welchem unter anderem auch Kalzium gemessen werden kann. Eine weitere Vorrichtung sowie ein Verfahren zur extrakorporalen Blutreinigung ohne Citrat-Antikoagulation ist in der EP 1 175 917 offenbart.

In der DE 101 14 283 C2 wird ein Verfahren zur Ermittlung der Ionenkonzentration des Blutes eines Patienten bei der Citrat-antikoagulierten Hämodialyse und/oder Hämofiltration der eingangs genannten Art offenbart. Bei den Ionen handelt es sich vorzugsweise um Kalzium- und/ oder Magnesiumionen, wobei vorzugsweise Kalziumionen bestimmt werden. In Fig.1 ist eine für diese Zwecke geeignete Vorrichtung gezeigt, das auch aus der DE 10114 283 C2 bekannt geworden ist. Das Gerät weist einen Hämodialysator und/ oder Hämofilter 101 sowie einem extrakorporalen Blutkreislauf 102 auf, der einen arteriellen Zulauf 103 (arterieller Schenkel) vom Patienten zum Hämodialysator und/oder Hämofilter 101 und einen venösen Ablauf 104 (venöser Schenkel) vom Hämodialysator und/oder Hämofilter 101 zum Patienten beinhaltet. Im Zulauf 103 befindet sich eine Citrat-Zufuhreinrichtung 105 und im Ablauf 104 eine Substitutionsmedium-Zufuhreinrichtung 106. Weiters hat das Gerät eine Dialysatleitung 107, welche wiederum einen Dialysatzulauf 108 und einem Dialysatablauf 109 aufweist. In diesem Verfahren wird die Ionenkonzentration des Blutes indirekt anhand der Ionenkonzentration im Dialysatablauf 109 bestimmt. Zu diesem Zwecke befindet sich im Dialysatablauf 109 zumindest ein Mittel zur Erfassung einer Ionenkonzentration 110, welches z.B. ein ionensensitiver Sensor sein kann. Dieses Mittel zur Erfassung einer Ionenkonzentration 110 ist mit einer Regeleinheit sowie mit den Zufuhreinrichtungen für Citrat und/oder für das Substitutionsmedium verbunden. Um die Ionenkonzentration des Blutes mit dem in der DE 10114 283 C2 beschriebenen Verfahren ermitteln zu können, müssen die Ionen im Dialysat in unkomplexierter Form vorliegen. Eine Möglichkeit ist eine vorübergehende Unterbrechung der Citratinfusion in den Blutkreislauf. Die andere Möglichkeit ist die Freigabe des Ions aus dem Ion-Citrat-Komplex beispielsweise durch Änderung des pH-Werts durch Infusion von Säure 111 in den Dialysatablauf 109 stromauf des Mittels zur Erfassung einer Ionenkonzentration 110.

Der große Vorteil der Methode der DE 10114 283 C2 besteht darin, dass für die Ermittlung der Ionenkonzentration des Blutes kein Eingriff in den blutseitigen Teil des extrakorporalen Schlauchsystems vonnöten ist. Diesem stehen folgende Nachteile gegenüber. Wie oben bereits erwähnt, ist eine sehr engmaschige und vorteilhafterweise kontinuierliche sowie möglichst genaue Bestimmung der Ionenkonzentration des Patientenblutes für einen die Sicherheit gewährleistenden Ablauf der Citrat-Antikoagulation notwendig. Eine kurzzeitige Unterbrechung der Citratzugabe zur Bestimmung der Ionenkonzentration des Blutes gemäß der einen in der DE 101 14 283 C2 beschriebenen Vorgangsweise ermöglicht demnach nur eine diskontinuierliche Überwachung der Ionenkonzentration und folglich eine diskontinuierliche Steuerung der Antikoagulation. Selbst bei einer kurzfristigen Unterbrechung der Citratzufuhr kann nicht gänzlich gesichert werden, dass eine ausreichende Antikoagulation gewährleistet werden kann.

Wird gemäß der anderen Vorgangsweise in DE 101 14 283 C2 die Ionenkonzentration des Blutes über die Ionenkonzentration des Dialysats durch Freisetzung des Ions aus dem Ion-Citrat-Komplex bestimmt, ist keine Unterbrechung der Citratzufuhr notwendig. Stark limitierend für diese Ausführungsform ist der verhältnismäßig große Aufwand und vor allem die Schwierigkeit, die Ionenkonzentration des Blutes genauer zu bestimmen, da dies aufgrund der geringeren Clearance des Ion-Citrat-Kömplexes im Vergleich zu freien Ionen und einer möglicherweise nicht vollständigen Freigabe der komplexierten Ionen schlichtweg nicht möglich ist. Eine ungenaue Bestimmung der Ionenkonzentration birgt daher das große Risiko einer Falschdosierung der Infusionslösungen. Zur Bestimmung der Ionenkonzentration des Blutes anhand der Konzentration im Dialysat muss gemäß dem in DE 10114 283 C2 offenbarten Verfahren der Blut- und Dialysatfluss miteinbezogen werden, da der Dialysatfluss während der Behandlung üblicherweise größer als der Blutfluss ist und somit die Ionenkonzentration des Blutes nicht derjenigen des Dialysats entspricht. Die Bestimmung der Ionenkonzentration des Blutes kann entweder rechnerisch erfolgen, was aber keine genaue Bestimmung zulässt, oder vorteilhafterweise - denn nur dann ist eine genauere Bestimmung möglich - durch Reduktion des Dialysatflusses und eine damit einhergehende Angleichung der Ionenkonzentration des Dialysats an jene des Blutes. Eine Erniedrigung des Dialysatflusses bringt jedoch den Nachteil mit sich, dass die Dialyseeffektivität während dieser Zeit erniedrigt ist. Hier besteht bei gewissen Patientengruppen, z.B. Patienten mit Lebererkrankungen, das Risiko einer intrakorporalen Citratakkumulation, wenn bei fortlaufender Citratinfusion der Dialysatfluss und somit auch die effektive Citratclearance reduziert werden. Darüber hinaus verlängert eine Reduktion des Dialysatflusses die Dialysedauer für den Patienten und hat, wirtschaftlich gesehen, einen vermehrten Einsatz von Ressourcen zur Folge. Somit ist eine genaue und gleichzeitig sehr engmaschige oder vorteilhafterweise kontinuierliche Bestimmung der Ionenkonzentration des Blutes bei kontinuierlich fortlaufender Dialyse mit dem Verfahren in DE 101 14 283 C2 nicht durchführbar. Zusätzlich gestaltet sich eine Automatisierung und Standardisierung der Citrat-Antikoagulation aufgrund der Komplexität des Systems schwierig.

Zusammenfassend können die gegenwärtigen Probleme der Citrat-Antikoagulation bei der klinischen Anwendung mit dem oben zitierten Stand der Technik nicht zufriedenstellend gelöst werden.

Die Erfindung ist im Rahmen eines Forschungsprojektes unter Beteiligung des Zentrums für Biomedizinische Technologie der Donau-Universität Krems entstanden. Das Ziel des Projekts ist die Entwicklung eines automatisierten Online- Citrat- Kalzium Antikoagulationssystems für eine einfache und sichere Anwendung. "Online" bedeutet eine kontinuierliche Regelung des Kalziumspiegels des Patienten mit Hilfe eines Sensors und einer Anpassung der Citrat- und Kalziuminfusionsraten. Die Kalziuminfusion wird dabei von einem Kalziumsensor geregelt. Dafür wurde ein eigenes Gerät - ein "Zitrat-Kalzium-Monitor" - entwickelt, welches im Wesentlichen zwei Infusionspumpen beinhaltet. Mittels des "Zitrat-Kalzium-Monitors" kann die Citrat- und Kalziuminfusion gezielt gesteuert werden. Ein spezieller Algorithmus berechnet die Kalziuminfusionsrate in Abhängigkeit der Blutflussrate und dem gemessenem ionisiertem Kalzium im Patienten. Eine Beschreibung eines "Zitrat-Kalzium-Monitors" ohne Kalzium-Sensor ist in [Schrefl A, Kellner KH, Hartmann J, Strobl W, Falkenhagen D (2001), A novel monitor for Anticoagulation with citrate. Int. J. Artif. Organs 24 / 8, 15] beschrieben.

Die Aufgabe der hier vorgestellten Erfindung ist die Verbesserung der Erfassung der Ionenkonzentration des Blutes bei der Durchführung und Überwachung der lokalen Citrat-antikoagulierten extrakorporalen Blutreinigung. Die Patientensicherheit steht dabei im Vordergrund. Um diese zu gewährleisten, ist eine zuverlässige Ermittlung und sorgfältige Überwachung spezifischer Blutparameter - vorwiegend die Kalziumionenkonzentration des Blutes - und eine dadurch ermöglichte strikte und automatisierte Regelung der Zufuhrraten des Citrats und des Substitutionsmediums von größter Wichtigkeit.

Diese Aufgabe wird mit einer Vorrichtung der eingangs genannten Art gelöst, welche erfindungsgemäß durch die Merkmale des Kennzeichens des Anspruchs 1 gekennzeichnet ist.

Außerdem wird ein Verfahren beschrieben, bei welchem die Ionenkonzentration in einem Blutzulauf des extrakorporalen Blutkreislaufes zwischen Patient und Dialyseeinheit zumindest an einer Stelle stromauf der Citratzufuhrstelle laufend gemessen wird, wobei die Steuerung der Dosierung des Citrats und des Substitutionsmediums unter Berücksichtigung eines stromab der Citratzufuhrstelle vorgebbaren Zielwerts oder Zielwertbereichs laufend durchgeführt wird.

Dank der Erfindung können die Probleme, die für den Stand der Technik spezifisch sind, auf einfache Art und Weise gelöst werden. Die Erfindung bringt besonders große Vorteile hinsichtlich der Patientensicherheit mit sich. Durch die laufende Messwerterfassung kann die Dosierung des Substitutionsmediums in sehr kleinen Zeitintervallen eingestellt werden. Die Dosierung des Citrats und des Substitutionsmediums erfolgt unter Berücksichtigung eines stromab der Citratzufuhrstelle vorgebbaren Zielwerts oder Zielwertbereichs. Der Zielwert oder Zielwertbereich ist in der Steuerung vorgebbar und durch den Benutzer einstellbar. Der Zielwert oder Zielwertbereich kann während der Blutreinigung bei Bedarf jederzeit verändert oder neu eingestellt werden. Die Erfindung ermöglicht somit eine laufende Durchführung und Überwachung der Antikoagulation und der Substitution. Stromauf der Zugabestelle für Citrat liegen die Ca- und Mg-Ionen noch in unkomplexierter Form vor und können quantitativ - und natürlich auch qualitativ - bestimmt werden, ohne dass es wie im oben zitierten Stand der Technik einer Unterbindung des Ion-Citrat-Komplexes bedarf. Der laufend ermittelte Messwert an dieser Position im Blutkreislauf stellt die laufend ermittelte "aktuelle Ionenkonzentration des Blutes" dar, also jene vor der Antikoagulation und der Blutreinigung, und spiegelt folglich den aktuellen intrakorporalen physiologischen Zustand des Patienten zum Zeitpunkt der Messung wider. Das Substitutionsmedium wird in Abhängigkeit eines Vergleichs zwischen einem Sollwertbereich und der gemessenen Ionenkonzentration dosiert. Statt eines Sollwertbereichs kann auch ein Sollwert eingestellt werden.

Durch die laufende Überwachung der Ionenkonzentration - vorzugsweise der Ca-Ionenkonzentration - des Blutes an dieser Position, kann auf einen Trend in Richtung eines unerwünschten physiologischen Zustands vom System frühzeitig und sehr schnell reagiert werden. Beispielsweise wird dadurch eine sehr frühzeitige Erkennung der Entwicklung einer Hypokalzämie beim Patienten ermöglicht. Eine Hypokalzämie kann bei gewissen Patientengruppen sehr schnell auftreten und schon innerhalb von kurzer Zeit eine lebensgefährliche Komplikation darstellen. Weiters ist durch die Messung der Ionenkonzentration des Blutes im blutseitigen Teil des extrakorporalen Kreislaufes im Vergleich zum Stand der Technik nicht nur eine sehr engmaschige oder kontinuierliche, sondern gleichzeitig - in Abhängigkeit des verwendeten Sensors - auch eine sehr genaue Quantifizierung der Ionen möglich. Ein weiterer großer Vorteil dieses Verfahrens hinsichtlich der Patientensicherheit ergibt sich daraus, dass eine laufende und akkurate Messwerterfassung im Gegensatz zum Stand der Technik im Wesentlichen ohne eine messtechnisch bedingte Auswirkung auf die regionale Antikoagulation und/oder Dialyseeffektivität durchgeführt werden kann. Aufgrund der im Vergleich zum oben zitierten Stand der Technik geringen Komplexität der Steuerung des erfindungsgemäßen Verfahrens kann die Citrat-Antikoagulation benutzerfreundlich und automatisiert durchgeführt werden.

Der Grundgedanke der Erfindung ist eine laufend durchgeführte Messung der Ionenkonzentration des Blutes und eine damit einhergehende laufend durchgeführte Regelung der Citrat-Antikoagulation, die dank der Erfindung automatisiert durchgeführt werden kann. Die Ionenkonzentration des Blutes wird dabei nicht wie im oben zitierten Stand der Technik indirekt anhand der Ionenkonzentration im Dialysat, sondern direkt blutseitig im extrakorporalen Blutkreislauf ermittelt.

Unter dem Begriff "laufend" wird erfindungsgemäß eine Messung der Ionenkonzentration des Blutes in regelmäßigen Zeitabständen verstanden, wobei die Zeitabstände vorteilhafterweise ausreichend kurz gehalten werden, um die Ionenkonzentration ausreichend engmaschig zu überwachen. Ausreichend kurze Zeitabstände sind solche, bei denen gewährleistet werden kann, dass eine Entwicklung in Richtung eines unphysiologischen Zustands des Patienten rechtzeitig erkannt und dieser entgegengewirkt werden kann.

Die Weiterbildungen des erfindungsgemäßen Verfahrens entsprechen den Weiterbildungen der erfindungsgemäßen Vorrichtung.

Aufgrund der bereits erwähnten Bedeutung der Kalzium- und Magnesiumionen bei physiologischen Abläufen im menschlichen Körper ist es zweckmäßig, wenn das Ionenkonzentrationsmessmittel Erdalkaliionen misst.

In Hinblick auf die Signifikanz des Kalziums bei der Antikoagulation ist es für die praktische Anwendung der Erfindung von Vorteil, wenn das Ionenkonzentrationsmessmittel Kalziumionen misst.

Für die Erfindung ist es weiters von Vorteil, wenn das Ionenkonzentrationsmessmittel ein ionensensitiver Sensor ist.

Um sowohl den Aufwand als auch die Kosten der Messung niedrig zu halten, ist es besonders praxistauglich, wenn das Ionenkonzentrationsmessmittel ein optischer ionensensitiver Sensor ist.

Bei der praktischen Durchführung hat sich eine Ausführung für zweckmäßig erwiesen, bei welcher das Ionenkonzentrationsmessmittel ein auf Fluoreszenz basierender optischer ionensensitiver Sensor ist.

Bei der Erfindung ist es vorgesehen, dass das Ionenkonzentrationsmessmittel in den extrakorporalen Blutkreislauf eingesetzt ist. Bei dieser Ausgestaltung findet die Messung im blutseitigen Teil des extrakorporalen Kreislaufes statt und das Ionenkonzentrationsmessmittel - vorzugsweise ein ionensensitiver Sensor - steht in Kontakt mit dem vorbeiströmenden Blut. Daher ergeben sich hohe Ansprüche an die Beschaffenheit des Sensors, da dieser den Forderungen nach Sterilität, Biokompatibilität und Wirtschaftlichkeit entsprechen muss. Unter dem Begriff "Biokompatibilität" wird im Sinne der Erfindung eine biologische Verträglichkeit zwischen einem Material - erfindungsgemäß die Sensorfläche - und einem Biosystem - erfindungsgemäß das Blut - verstanden. Gleichzeitig besteht die Notwendigkeit, dass mittels des Sensors genaue und zuverlässige Konzentrationswerte laufend erfasst werden. Die Genauigkeit der erhaltenen Messwerte hängt vom verwendeten Sensor ab. Aus dem Stand der Technik sind bereits Sensoren bekannt, die die oben genannten Anforderungen erfüllen und sind beispielsweise in der WO 2006/029293 und der US 4,344,438 beschrieben. Die in der WO 2006/029293 beschriebene Sensorvorrichtung ist unter anderem besonders für extrakorporale Blutkreisläufe vorgesehen. Besonders von Vorteil sind auch Verfahren und Sensorvorrichtungen, die in den Blutstrom eingesetzt werden und die Konzentration spezifischer Komponenten anhand ihrer optischen Eigenschaften erfassen. Eine Beschreibung für ein solches Verfahren und eine Sensorvorrichtung findet sich beispielsweise in der US 4,344,438.

Gemäß einer weiteren vorteilhaften Ausgestaltung kann vorgesehen sein, dass eine geringe Menge Blut über zumindest eine Bypass-Leitung vom extrakorporalen Blutkreislauf abgezweigt wird und in dieser Bypass-Leitung zumindest ein Ionenkonzentrationsmessmittel angeordnet ist. Die Anforderungen in Bezug auf die Biokompatibilität sind bei dieser Ausgestaltung nicht limitierend, da die geringe Menge abgezweigten Blutes nach der Messung nicht mehr in den extrakorporalen Blutkreislauf zurückgeführt, sondern verworfen wird.

In Hinblick auf die Patientensicherheit kann die erfindungsgemäße Vorrichtung weiters zweckmäßigerweise mit zusätzlichen Ionenkonzentrationsmessmitteln versehen werden:

So kann vorgesehen sein, dass ein zusätzliches Ionenkonzentrationsmessmittel stromab der Citratzufuhrstelle und stromauf der Dialyseeinheit angeordnet ist. Erfindungsgemäß findet dabei eine zusätzliche Messung der Ionenkonzentration stromab der Citratzufuhrstelle und stromauf der Dialyseeinheit statt, wobei die gemessene Ionenkonzentration eine Regelgröße für die Citratzufuhrrate ist und als Istwert mit einem Sollwert und/oder Sollwertbereich verglichen wird und ein Überschreiten des Sollwerts und/oder Sollwertbereichs eine Erhöhung der Citratzufuhrrate und ein Unterschreiten des Sollwerts und/oder Sollwertbereichs eine Erniedrigung der Citratzufuhrrate bewirkt.

In einer weiteren Variante kann vorgesehen sein, dass ein zusätzliches Ionenkonzentrationsmessmittel stromab der Dialyseeinheit und stromauf der Substitutionsmedium-Zufuhrstelle angeordnet ist. Somit kann eine zusätzliche Messung der Ionenkonzentration stromab der Dialyseeinheit und stromauf der Substitutionsmedium-Zufuhrstelle durchgeführt werden, wobei die gemessene Ionenkonzentration eine Regelgröße für die Substitutionsmediumszufuhrrate ist.

Gemäß einer weiteren Variante kann vorgesehen sein, dass ein zusätzliches Ionenkonzentrationsmessmittel stromab der Substitutionsmedium-Zufuhrstelle vorgesehen ist. Bei dieser Variante findet eine zusätzliche Messung der Ionenkonzentration stromab der Substitutionsmedium-Zufuhrstelle statt, wobei die gemessene Ionenkonzentration eine Regelgröße für die Substitutionsmediumszufuhrrate ist und als Istwert mit einem Sollwertbereich verglichen wird, wobei ein Überschreiten des Sollwertbereichs eine Verminderung der Substitutionsmediums-Zufuhrrate und ein Unterschreiten des Sollwertbereichs eine Erhöhung der Substitutionsmediumszufuhrrate bewirkt.

Weiters besteht im Rahmen der Erfindung die Möglichkeit, dass in einem Plasmakreislauf ein zusätzliches Ionenkonzentrationsmessmittel angeordnet ist. Bei dieser Variante kann die Ionenkonzentration zusätzlich im Plasmakreislauf gemessen werden, wobei die gemessene Ionenkonzentration eine Regelgröße für die Citratzufuhrrate ist und als Istwert mit einem Sollwert und/oder Sollwertbereich verglichen wird und ein Überschreiten des Sollwerts und/oder Sollwertbereichs eine Erhöhung der Citratzufuhrrate bewirkt und ein Unterschreiten des Sollwerts und/oder Sollwertbereichs eine Reduktion der Citratzufuhrrate bewirkt. Dies ist nur von Vorteil, wenn der Plasmakreislauf geschlossen ist, da sich nur dann die Ionenkonzentration im Plasmakreislauf so einstellt, dass zuverlässige Messwerte erhalten werden können.

Liefert eines der Ionenkonzentrationsmessmittel einen Messwert außerhalb eines vordefinierbaren Sollwert- und/ oder Zielwertbereichs, so kann ein Alarm ausgelöst und die Zufuhr des Citrats und des Substitutionsmediums gestoppt werden. Jeglicher Alarm der Dosiereinrichtungen kann zu einer Alarmierung des Blutreinigungssystems führen und führt dieses in einen sicheren Zustand. Dieser Alarm kann beispielsweise in Form eines akustischen und/oder optischen Signals erfolgen.

Um die Citrat-Antikoagulation automatisiert durchführen zu können und die Patientensicherheit hoch zu halten, ist es zweckmäßig, wenn die Dosiereinrichtungen für Citrat und für das Substitutionsmedium und das extrakorporale Blutreinigungssystem über die Steuerung mittels Signalverbindungen miteinander verbunden sind, wobei die Steuerung dazu eingerichtet ist, ihr zugeführte Signale des extrakorporalen Blutreinigungssystems in ihre Regelung einzubeziehen. Signale des extrakorporalen Blutkreislaufes können beispielsweise eine Änderung der Höhe des extrakorporalen Blutflusses oder des Dialysatflusses, die Art des verwendeten Dialysators/Hämofilters, die eingestellte Ultrafiltrationsrate, ein Stoppen der Blutpumpe oder ein Alarm, welcher den Stillstand des extrakorporalen Blutflusses bewirkt, sein. Die Höhe des extrakorporalen Blutflusses [ml/min] ist das Blutvolumen, welches pro Zeiteinheit von der Blutpumpe gepumpt wird.

Weiters ist es von Vorteil, wenn die Dosiereinrichtungen für Citrat und für das Substitutionsmedium und das extrakorporale Blutreinigungssystem über die Steuerung mittels Signalverbindungen miteinander verbunden sind, wobei die Steuerung dazu eingerichtet ist, ihr zugeführte Signale der Dosiereinrichtungen in ihre Regelung einzubeziehen.

Solche Signale der Dosiereinrichtungen können beispielsweise eine verbrauchte Citratlösung bzw. ein verbrauchtes Substitutionsmedium, ein abgeklemmten Schlauch oder die Anwesenheit von Luftblasen im Schlauch anzeigen.

Darüber hinaus ist es von Vorteil, wenn bei einer Filtrationseinrichtung des extrakorporalen Blutreinigungssystems, wobei die Dosiereinrichtungen für Citrat und für das Substitutionsmedium und das extrakorporale Blutreinigungssystem über die Steuerung mittels Signalverbindungen miteinander verbunden sind, die Steuerung dazu eingerichtet ist, bei einer Änderung der Citratzufuhrrate und/oder der Substitutionsmediumszufuhrrate eine proportionale Änderung der Ultrafiltrationsmenge der Filtrationseinrichtung zu veranlassen oder einem Bediener vorzuschlagen. Im Zuge der Dialysebehandlung kann es, wie bereits erwähnt, vorgesehen sein, dass dem Patienten Flüssigkeit entzogen wird (Ultrafiltration). Der Beitrag der Ultrafiltration zur Blutreinigung ist unwesentlich und dient allein zur Entfernung von Flüssigkeitsansammlungen im Patienten. Ist die verwendete Citratlösung bzw. die Substitutionsmediumlösung sehr niedrig konzentriert, so gelangt im Zuge der Zuführung des Citrats bzw. des Substitutionsmediums ein größeres Flüssigkeitsvolumen in den extrakorporalen Blutkreislauf und somit in den Patienten, welches durch Ultrafiltration wieder entfernt werden muss. Bei der Ultrafiltration kann es weiters zu einer Änderung des Hämatokrits kommen. Der Hämatokrit ist, wie weiter unten im Detail erläutert, ein wichtiger Parameter, welcher bei der Zufuhr des Citrats und/oder des Substitutionsmediums berücksichtigt werden sollte.

Um die Patientensicherheit möglichst hoch zu halten ist es zweckmäßig, wenn die Ionenkonzentration laufend in regelmäßigen Zeitabständen gemessen wird. Die Zeitintervalle sollen möglichst kurz gehalten werden, sodass die Überwachung und Steuerung der Citrat-Antikoagulation möglichst engmaschig erfolgt. Bei den meisten Anwendungen ist ein Zeitintervall von 30 - 60 Minuten ausreichend.

Bei gewissen Patientengruppen, wie z.B. Patienten mit Lebererkrankungen oder Personen mit geringem Körpergewicht (vor allem Kinder) ist es ratsam, wenn der Zeitabstand zwischen zwei aufeinanderfolgenden Messungen maximal 10 Minuten beträgt.

Um eine möglichst engmaschige oder kontinuierliche Erfassung der Ionenkonzentration zu ermöglichen, ist es von Vorteil, wenn mittels eines einzigen Ionenkonzentrationsmessmittels in regelmäßigen Zeitabständen ein Messsignal generiert wird. Dem Ionenkonzentrationsmessmittel oder Sensorsystem entsprechend, kann der Sensor entweder ohne oder mit Unterbrechung arbeiten. Der Sensor kann während der Unterbrechung rekalibriert werden.

Eine engmaschige Messwerterfassung wird auch durch eine Variante ermöglicht, bei welcher die Ionenkonzentration von zwei oder mehreren wesensgleichen Ionenkonzentrationsmessmitteln alternierend bzw. turnusmäßig gemessen wird, wobei in regelmäßigen Zeitabständen ein Messsignal generiert wird. Bei dieser Variante können die Messungen sehr engmaschig durchgeführt werden, da eine Rekalibrierung des jeweils momentan nicht messenden Ionenkonzentrationsmessmittels beispielsweise durch kurzzeitiges Spülen mit einer Kalibrierlösung möglich ist.

Zusätzlich zur Quantifizierung der Ionen ist es für den Fachmann einsichtig, dass im Rahmen der Erfindung auch vorgesehen sein kann, dass die Ionen nicht nur quantitativ, sondern auch qualitativ bestimmt werden können. Anstelle von Sensoren können natürlich auch andere Verfahren zur Bestimmung der Ionenkonzentration, beispielsweise eine Messung mittels Ionometer, eingesetzt werden.

Um die Dosierung der Citratzufuhr einzustellen und die Citratzufuhrrate zu regeln, ist es für die Erfindung von Vorteil, wenn die initiale Kalziumionenkonzentration und der Hämatokrit als Parameter bei der Dosierung des Citrats und der Substitutionslösung berücksichtigt werden und die Höhe des extrakorporalen Blutflusses eine Regelgröße für die Citratzufuhrrate ist, wobei eine Änderung der Höhe des extrakorporalen Blutflusses eine proportionale Änderung der Citratzufuhrrate bewirkt. Die Citratzufuhrrate ist die Menge an Citrat, die pro Zeiteinheit an einer Citratzufuhrstelle in den extrakorporalen Blutkreislauf eingebracht wird. Die Citratzufuhrrate wird dabei so eingestellt und im Laufe der Behandlung so geregelt, dass eine effektive Antikoagulation gewährleistet ist. Um die Citratzufuhrrate einzustellen, werden erfindungsgemäß verschiedene Parameter berücksichtigt:

Die initiale Kalziumionenkonzentration des Patienten ist jene Kalziumionenkonzentration, die einmalig vor Beginn der Blutreinigung bestimmt wird. Ausgehend von diesem Wert wird die Citratzufuhrrate zu Beginn der Behandlung eingestellt.

Um die verabreichte Citratdosis den individuellen Bedürfnissen des Patienten anzupassen, wird weiters der Hämatokrit als Parameter bei der Einstellung der benötigten Citratdosis berücksichtigt. Die Menge an Citrat, die für eine effektive Antikoagulation notwendig ist, wird vom Hämatokrit beeinflusst. Der Grund dafür ist die Tatsache, dass Citrat nicht durch die Zellmembranen der Blutzellen permeieren kann und sich daher nur im Plasma verteilt. Bei Patienten mit niedrigem Hämatokrit ist folglich eine höhere Citratdosis für eine effektive Antikoagulation notwendig und vice versa. Der Hämatokrit wird vor Beginn der Behandlung einmalig bestimmt, stellt also den initialen Hämatokrit dar und wird bei der Citratzufuhrrate als konstanter Faktor berücksichtigt. Natürlich wird ein Fachmann auf dem Gebiet erkennen, dass es im Sinne der Erfindung möglich ist, den Hämatokrit nicht nur eingangs, sondern während des gesamten Dialysevorgangs in Zeitabständen erneut zu ermitteln und die Citratzugabe den Hämatokritwerten entsprechend anzupassen.

Um die Dosierung des Citrats und somit die lokale Antikoagulation so akkurat wie möglich zu halten, ist es weiters zweckmäßig, wenn die Höhe des extrakorporalen Blutflusses eine Regelgröße für die Citratzufuhrrate ist, wobei eine Änderung der Höhe des extrakorporalen Blutflusses eine proportionale Änderung der Citratzufuhrrate bewirkt. Die Citratzufuhrrate ist mit dem extrakorporalen Blutfluss über einen konstanten Faktor gekoppelt, sodass eine Änderung des extrakorporalen Blutflusses eine Änderung der Citratzufuhrrate bewirkt. Bei einer Erhöhung des Blutflusses wird die Citratzufuhrrate ebenfalls erhöht. Bei einem Anhalten des Blutflusses, beispielsweise ausgelöst durch einen Ausfall der Blutpumpe, stoppt daher auch die Citratzufuhr.

Die Dosierung des Citrats kann sehr genau eingestellt werden, wenn weiters die Bindung der Kalziumionen an im Blut vorkommende Proteine als Parameter bei der Dosierung des Citrats berücksichtigt wird. Die Bindung der Kalziumionen an Citrat wird durch die im Blut vorkommenden Proteine, insbesondere Albumin, beeinflusst. Es kommt zur Bildung von Protein-Kalzium-Komplexen. Für die Berechnung, wie viele Kalziumionen proteingebunden vorliegen, kann das Massenwirkungsgesetz herangezogen werden. Die Citratzufuhrrate wird unter Berücksichtigung dieses Parameters zu Beginn der Behandlung eingestellt.

Durch Zugabe des Citrats wird die Kalziumionenkonzentration auf einen gewünschten Zielwert und/oder Zielwertbereich abgesenkt. Für das erfindungsgemäße Verfahren ist es daher günstig, wenn der stromab der Citratzufuhrstelle vorgebbare Zielwert und/oder Zielwertbereich die Kalziumionenkonzentration des antikoagulierten Blutes, welche zwischen der Citratzufuhrstelle und der Dialyseeinheit vorliegt, repräsentiert. Der Zielwert und/oder Zielwertbereich ist sinnvollerweise jene Kalziumionenkonzentration, bei welcher eine Blutgerinnung im Filter effektiv verhindert wird. In der Literatur finden sich dabei Zielwerte, die als Grenzwert der Kalziumionenkonzentration im antikoagulierten Blut Werte im Bereich von 0,25 bis 0,35 mmol/l [Kutsogiannis et al. (2000), Am J Kidney Dis, Vol. 35:802-811; Tolwani et al. (2001), Kidney International, Vol. 60:370-374] bzw. < 0,4 mmol/l [Swartz et al. (2004), Clinical Nephrology, Vol. 61(2):134-143] angeben. Innerhalb dieses Bereiches soll die Kalziumionenkonzentration geregelt werden.

Für die Patientensicherheit ist es dabei besonders empfehlenswert, wenn die Kalziumionenkonzentration des antikoagulierten Blutes in einem Bereich zwischen 0,15 - 0,5 mmol/l, und insbesondere zwischen 0,2 - 0,4 mmol/l wählbar ist. Der Zielwert kann entsprechend dem Zustand und den Bedürfnissen des Patienten individuell gewählt werden. So kann es bei manchen Patienten notwendig sein, den Zielwert auf 0,15 mmol/l einzustellen, während bei anderen Patienten ein höherer Zielwert von bis zu 0,5 mmol/l optimal ist. Idealerweise ist ein Wert von ca. 0,2 mmol/l anzustreben, da bei diesem Wert auch die Komplementaktivierung stark reduziert ist. Dieser Zielwert und/oder Zielwertbereich wird zu Beginn der Behandlung vorgegeben und ist durch den Benutzer einstellbar. Der Zielwert und/oder Zielwertbereich kann während der Blutreinigung bei Bedarf verändert und neu eingestellt werden.

Über- oder unterschreitet die stromauf der Citratzufuhrstelle gemessene Ionenkonzentration einen zuvor definierten Sollwertbereich, so kann es vorgesehen sein, dass die stromauf der Citratzufuhrstelle gemessene Ionenkonzentration eine Regelgröße für die Citratzufuhrrate ist, wobei die gemessene Ionenkonzentration als Istwert mit einem Sollwertbereich verglichen wird und ein Überschreiten des Sollwertbereiches eine Erhöhung der Citratzufuhrrate und ein Unterschreiten des Sollwertbereiches eine Erniedrigung der Citratzufuhrrate bewirkt. Wie bereits erwähnt spiegelt die stromauf der Citratzufuhrstelle gemessene Ionenkonzentration - vorzugsweise die Kalziumionenkonzentration - den aktuellen intrakorporalen physiologischen Zustand des Patienten wieder. Der Sollwertbereich umfasst daher Ionenkonzentrationswerte, die einem physiologischen Zustand entsprechen. Selbstverständlich kann Unter- oder Überschreiten des Sollwertbereiches auch einen Alarm auslösen.

Um die Dosierung des Substitutionsmediums zur Aufrechterhaltung des physiologischen Zustands des Patienten optimal einzustellen, ist es für die praktische Anwendung zweckmäßig, wenn die stromauf der Citratzufuhrstelle gemessene Ionenkonzentration eine Regelgröße für die Substitutionsmediumszufuhrrate ist, wobei die gemessene Ionenkonzentration als Istwert mit einem Sollwertbereich verglichen wird und ein Überschreiten des Sollwertbereiches eine Erniedrigung der Substitutionsmediumszufuhrrate und ein Unterschreiten des Sollwertbereiches eine Erhöhung der Substitutionsmediumszufuhrrate bewirkt.

Weiters kann die Citrat-Clearance und/oder die Kalziumionen-Clearance der Dialyseeinheit eine Regelgröße für die Substitutionsmediumszufuhrrate sein, wobei eine Erhöhung der Citrat-Clearance bzw. eine Erniedrigung der Kalziumionen-Clearance eine Erniedrigung der Substitutionsmediumszufuhrrate bewirkt, und eine Erniedrigung der Citrat-Clearance bzw. eine Erhöhung der Kalziumionen-Clearance eine Erhöhung der Substitutionsmediumszufuhrrate bewirkt. Das Ausmaß der Entfernung des Citrats, der Ion-Citrat-Komplexe bzw. der Kalziumionen durch den Dialysefilter (Citrat-Clearance bzw. Kalziumionen-Clearance) ist abhängig vom verwendeten Filter sowie vom Blut- und Dialysatfluss bzw. dem Verhältnis von Blut zu Dialysatfluss. Folglich ist es ratsam, wenn die Citrat-Clearance und/oder die Kalziumionen-Clearance der Dialyseeinheit bei der Substitution der Ca- und eventuell Mg-Ionen berücksichtigt werden. Das Substitutionsmedium, welches stromab des Dialysefilters in den extrakorporalen Kreislauf eingebracht wird, beinhaltet üblicherweise vorwiegend Ca-Ionen (bzw. zusätzlich auch Mg-Ionen).

Die zu erwartende Citrat-Clearance und/oder Kalziumionen-Clearance kann dem Citrat-Antikoagulationssystem kommuniziert werden, indem vor Beginn der Behandlung dem System der eingesetzte Filter durch den Bediener eingegeben wird. Alternativ kann die Clearance durch die Eingabe des Filtertyps (High Flux / Low Flux) und der effektiven Membranfläche kommuniziert werden. Für die Bestimmung der Citrat-Clearance bzw. der Kalziumionen-Clearance ist es wesentlich, den Blut- und den Dialysafluss an den Citrat-Monitor zu kommunizieren.

Weiters ist es von großer Relevanz für die Patientensicherheit, wenn die Citratzufuhrrate bei der Regelung der Substitutionsmediumszufuhrrate berücksichtigt wird, wobei eine Erhöhung der Citratzufuhrrate eine Erhöhung der Substitutionsmediumszufuhrrate bewirkt und eine Erniedrigung der Citratzufuhrrate eine Erniedrigung der Substitutionsmediumszufuhrrate bewirkt. Wird die Citratzufuhr unterbrochen, beispielsweise durch einen Pumpenausfall, so wird auch die Zufuhr des Substitutionsmediums unterbrochen.

Im Folgenden wird die Erfindung samt weiteren Vorzügen anhand der beigefügten Zeichnungen und nichteinschränkender Ausführungsbeispiele näher erläutert. Die Zeichnungen zeigen:

Fig. 1    eine schematische Darstellung einer Citrat-antikoagulierten Blutreinigung, wie es aus der DE 10114 283 C2 bekannt ist,

Fig. 2    eine schematische Darstellung einer ersten bevorzugten Ausführungsform einer automatisierten Citratantikoagulierten Blutreinigung gemäß der Erfindung,

Fig. 3    ein Diagramm, welches die Citratkonzentration in Abhängigkeit der freien Kalziumionen und des Hämatokrits zeigt und zur Einstellung der Citratzufuhrrate herangezogen wird,

Fig. 4    eine schematische Darstellung einer zweiten, erweiterten Ausführungsform, und

Fig. 5    eine schematische Darstellung einer dritten Ausführungsform, die bei Fehlfunktionen einen Alarm auslöst.

Fig. 6a-e    Ausführungsbeispiele für Sensoren.

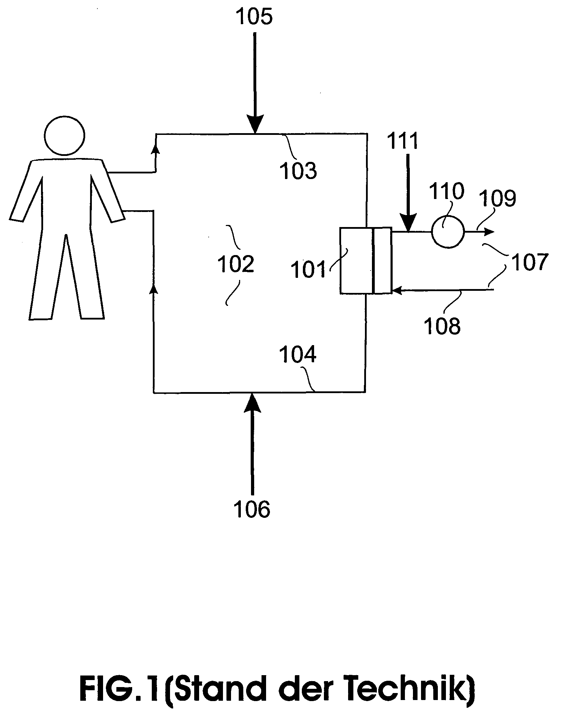

Das in Fig. 1 schematisch dargestellte Citrat-antikoagulierte Blutreinigungsverfahren gemäß der DE 101 14 283 wurde bereits weiter oben in der Beschreibung des Stands der Technik eingehend erläutert.

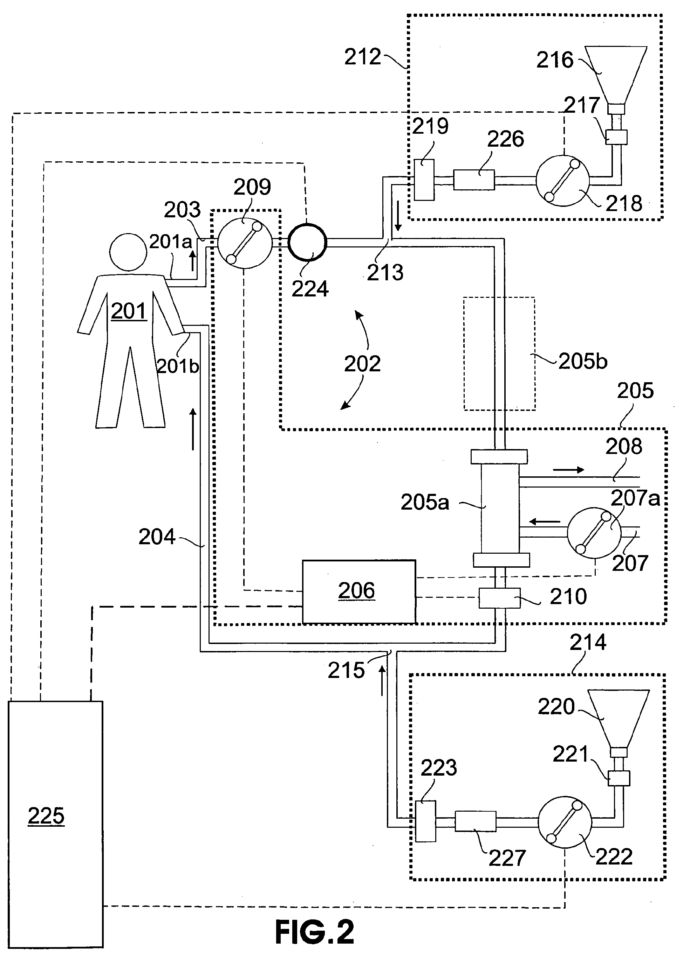

Die Signalverbindungen der einzelnen Vorrichtungsbauteile miteinander bzw. mit den Steuerungen sind in Fig. 2, 4 und 5 durch gestrichelte Linien symbolisiert.

Fig. 2 zeigt eine schematische Darstellung einer bevorzugten Ausführungsform eines automatisierten Citratantikoagulierten Blutreinigungsverfahrens gemäß der Erfindung. Das Blut des Patienten 201 gelangt über einen arteriellen Zugang 201a in den extrakorporalen Blutkreislauf 202 mit einem Blutzulauf 203 (arterieller Schenkel), einem Blutablauf 204 (venöser Schenkel) und einer dazwischen liegenden Dialyseeinheit 205 (durch eine gestrichelte Umrandung dargestellt), und fließt über einen venösen Zugang 201b wieder in den Patienten zurück. Die Dialyseeinheit 205 setzt sich im Wesentlichen aus einem Dialysefilter 205a, einer Blutpumpe 209, einer Luftfalle mit Luftblasendetektor 210 und einer Steuerung 206 zusammen. Je nach Blutreinigungssystem und erforderlicher Behandlung kann stromauf des Dialysefilters 205a noch ein zusätzliches Blutreinigungselement 205b - beispielsweise ein Hämofilter, ein Hämofilter mit geschlossenem Plasmakreislauf oder ein Adsorberkreislauf - vorgesehen sein. Im Dialysefilter 205a wird das Blut über eine semipermeable Membran mit der Dialysierflüssigkeit in Verbindung gebracht. Die Dialysierflüssigkeit wird üblicherweise vom Dialysegerät hergestellt und mittels einer Dialysatpumpe 207a über einen Dialysatzulauf 207 in den Dialysefilter 205a gepumpt und nach Durchlauf des Dialysefilters 205a über einen Dialysatablauf 208 abgeleitet und entsorgt. Das Blut wird vom Patienten kommend mittels der Blutpumpe 209 durch den extrakorporalen Blutkreislauf 202 gepumpt, wobei über die Blutpumpe 209 die Höhe des Blutflusses $Q_B$ eingestellt wird. Der Blutfluss $Q_B$ [ml/min] ist, wie weiter unten beschrieben, ein wichtiger Parameter für das System und stellt das Blutvolumen, das pro Zeiteinheit von der Pumpe gepumpt wird, dar. Typischerweise wird bei der Hämodialyse ein Blutfluss $Q_B$ = 50 - 400 ml/min eingestellt.

Bevor das Blut wieder in den Patienten 201 gelangt, passiert es noch eine Luftfalle mit Luftblasendetektor 210 zur Verhinderung einer Luftembolie. Die oben beschriebenen Bauteile 202 - 210 bilden eine typische Ausführung eines extrakorporalen Blutreinigungssystems.

Fig.2 zeigt weiters eine Vorrichtung zur Citrat-Antikoagulation mit einer gesteuerten Citrat-Dosiereinrichtung 212 zur Zuführung von Citrat an einer Citratzufuhrstelle 213 stromauf des Dialysefilters 205a (und des Blutreinigungselements 205b, wenn vorhanden) und einer gesteuerten Substitutionsmedium-Dosiereinrichtung 214 zur Zuführung eines Substitutionsmediums an einer Substitutionsmedium-Zufuhrstelle 215 stromab des Dialysefilters 205a.

Die Citrat-Dosiereinrichtung 212 weist einen Behälter mit Citratlösung 216, einen Tropfendetektor 217, eine Citratpumpe 218, einen Drucksensor 226 und einen Luftblasendetektor 219 auf. Durch die Infusion der Citratlösung, entweder in Form von $Na_3$-Citrat oder ACD-A (acid citrate dextrose-A), das neben Citrat auch Zitronensäure und Dextrose enthält, an der Citratzufuhrstelle 213 in den extrakorporalen Blutkreislauf 202, wird das Blut in seiner Gerinnung gehemmt. Ist die Citrat-Lösung, wie es beispielsweise bei einer ACD-A-Lösung der Fall ist - niedrig konzentriert, ergibt sich ein relativ großes Flüssigkeitsvolumen, welches pro Zeiteinheit in den extrakorporalen Blutkreislauf 202 gelangt. Aus diesem Grunde ist es notwendig, dies bei der Ultrafiltrationsmenge des Dialysegerätes zu berücksichtigen. Ähnlich der Citrat-Dosiereinrichtung 212, beinhaltet die Substitutionsmedium-Dosiereinrichtung 214 ebenfalls ein Behältnis mit Substitutionsmedium 220, einen Tropfendetektor 221, eine Substitutionsmediumspumpe 222, einen Drucksensor 227 und einen Luftblasendetektor 223. Das Substitutionsmedium enthält vorwiegend Ca-Ionen oder Ca- und Mg-Ionen und wird an der Subsitutionsmedium-Zufuhrstelle 215 in den extrakorporalen Blutkreislauf 202 infundiert. Eine Zufuhr des Substitutionsmediums über einen separaten venösen Zugang wäre ebenso möglich, ist aber aus Sicherheitsgründen nicht erwünscht.

Die Pumpendrehzahl der Pumpen 218, 222 wird mit in den Pumpen 218, 222 integrierten Hallsensoren und Inkrementalaufnehmern ermittelt und überwacht.

Die Messung der Patienten-Ionenkonzentration erfolgt mit einem stromauf der Citratzufuhrstelle 213 angeordneten Ionenkonzentrationsmessmittel, welches in der in Fig.2 gezeigten Ausführungsform ein Ca-Ionen-Sensor 224 ist, mit welchem sensitiv und spezifisch die Ca-Ionenkonzentration gemessen wird. Dabei ist es unwesentlich, ob der Ca-Ionen-Sensor 224 stromab oder stromauf der Blutpumpe 209 angeordnet ist. Für die Messung kann bei einer Variante eine geringe Menge Blut in regelmäßigen Zeitabständen vom extrakorporalen Blutkreislauf 202 abgezweigt und dem Ca-Ionen-Sensor 224 zugeführt werden, wobei das Blut nach der Messung verworfen wird. Selbstverständlich ist es auch möglich, einen biokompatiblen Ca-Ionen-Sensor 224 direkt in den extrakorporalen Blutkreislauf zu integrieren. Die aus der WO 2006/029293 bekannte Sensor-Vorrichtung könnte beispielsweise mit einer biokompatiblen Ca-Ionen-sensitiven Sensoroberfläche kombiniert werden.

Die Messungen werden in regelmäßigen Zeitabständen durchgeführt, wobei der Zeitabstand so gewählt wird, dass ein Trend in Richtung eines unphysiologischen Zustands des Patienten frühzeitig erkannt wird. Bei den meisten Anwendungen ist ein Zeitintervall von 30-60 Minuten ausreichend. Bei gewissen Patientengruppen, z.B. Patienten mit Leberererkrankungen, beträgt der Zeitabstand zwischen zwei aufeinanderfolgenden Messungen maximal 10 Minuten.

Die beiden Dosiereinrichtungen 212, 214 und der Ca-Ionen-Sensor 224 sind mit einer Steuerung 225 verbunden. Die Steuerung 225 ist weiters mit der Steuerung 206 der Dialyseeinheit 205 verbunden. Um das Citrat-antikoagulierte Blutreinigungsverfahren automatisiert durchzuführen, ist vorgesehen, dass die Steuerung 225, durch welche der Ca-Ionen-Sensor 224 und die beiden Dosiervorrichtungen 212, 214 verbunden sind, und die Steuerung der Dialyseeinheit (z.B. eine Fresenius 4008/5008 Dialysemaschine) über einen Steuerungsalgorithmus miteinander in Verbindung stehen. Die Steuerung 225 hat die Aufgaben, die Citrat- und Substitutionsmediumspumpen 218, 222 zu steuern und zu regeln, die Signale der Sensoren 224, 226, 227 und Detektoren 217, 219, 221, 223 auszuwerten, bei Fehlfunktionen Alarme auszulösen, die Pumpen 218, 222 bei Fehlfunktion abzuschalten, Daten zu speichern und mit dem Blutreinigungsgerät zu kommunizieren.

Zur Dosierung des Citrats und des Substitutionsmediums kann der an der Donau-Universtität Krems (Zentrum für Biomedizinische Technologie) entwickelte und im Stand der Technik zitierte "Zitrat-Kalzium Monitor" eingesetzt werden.

Um die Citrat-Antikoagulation zu steuern, wird ein einfacher Regelkreis geschaffen:

Die Citratzufuhrrate $Q_{cit}$ wird unter Berücksichtigung eines in der Steuerung 225 vorgebbaren Zielwertes eingestellt, wobei die initiale Ca-Ionenkonzentration des Blutes und der initiale Patientenhämatokrit bei der Einstellung der Citratzufuhrrate $Q_{cit}$ berücksichtigt werden und die Höhe des Blutflusses eine Regelgröße für die Citratzufuhrrate $Q_{cit}$ ist. Die initiale Ionenkonzentration des Blutes wird vor Beginn der Behandlung entweder mittels des Ca-Ionen-Sensors 224 oder mittels einer entnommenen Blutprobe bestimmt. Der initiale Hämatokrit wird ebenfalls vor Beginn der Behandlung anhand einer Blutprobe ermittelt. Der Zielwert und/oder Zielwertbereich repräsentiert jene Ca-Ionenkonzentration des antikoagulierten Blutes, welche stromab der Citratzufuhrstelle 213 und stromauf der Dialyseeinheit 205 vorliegt, und ist sinnvollerweise jene Ca-Ionenkonzentration, bei welcher eine Blutgerinnung im Filter effektiv verhindert wird. In der Literatur finden sich dabei Zielwerte, die eine Ca-Ionenkonzentration von 0,25 bis 0,4 mmol/l angeben. Idealerweise ist ein Wert von ca. 0,2 mmol/l anzustreben, da bei dieser Ca-Ionenkonzentration auch die Komplementaktivierung stark reduziert ist. Dieser Zielwert und/oder Zielwertbereich ist zu Beginn der Behandlung in der Steuerung 225 vorgebbar und durch den Benutzer einstellbar. Der Zielwert und/oder Zielwertbereich kann während der Blutreinigung bei Bedarf jederzeit verändert und neu eingestellt werden.

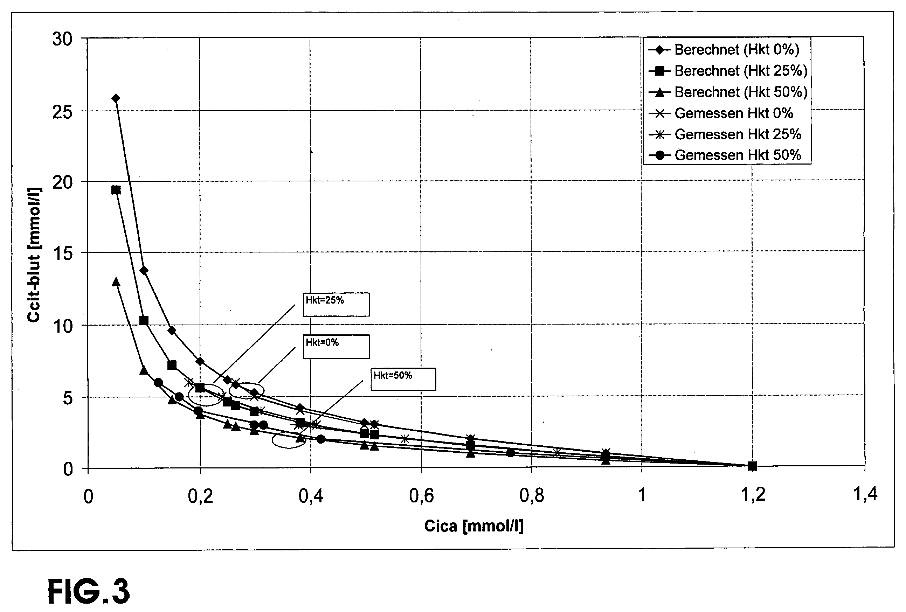

Die Einstellung der Citratzufuhrrate $Q_{cit}$ basiert auf einem mathematischen Modell, welches anhand des in Fig. 3 dargestellten Diagramms veranschaulicht wird.

Das Diagramm zeigt das Kennlinienfeld der Ca-Ionen-Absenkung mit Citrat, wobei die Citrat-Konzentration im Blut $C_{cit\_blut}$ [mmol/l] in Abhängigkeit der Zielkonzentration der freien Ca-Ionen $C_{iCa}$ [mmol/l] im antikoagulierten Blut und des initialen Hämatokrits Hkt [%] dargestellt ist. Die initiale Ca-Ionenkonzentration des Blutes liegt mit sehr geringen Abweichungen bei 1,2 mmol/l. Die Hämatokritwerte liegen meist in einem Bereich zwischen 25 und 50%. Die Kennlinien wurden - ausgehend von Hämatokritwerten von 0%, 25% und 50% - entweder durch Messung oder durch Berechnung erhalten.

Die Citrat-Konzentration im Blut $C_{cit-blut}$ zur Senkung der Ca-Ionen-Konzentration durch Komplexbildung errechnet sich dabei aus folgender empirischen Formel:

1) Berechnung der komplexgebundenen Kalziumionen:

$$C_{\text{Komplexgebundenes Ca}} = 1{,}2\,\text{mmol/l} - C_{iCa}$$

2) 1 mmol Kalzium bindet v mmol Citrat im Komplex:

$$C_{\text{Komplexgebundens Citrat}} = C_{\text{Komplexgebundenes Ca}} * v$$

3) Aus 1 und 2 ergibt sich:

$$C_{\text{freies Citrat}} = C_{cit\_blut} - (1{,}2\,\text{mmol/l} - C_{iCa}) * v$$

4) Aus dem Massenwirkungsgesetz ergibt sich:

$$C_{\text{freies Citrat}} * C_{iCa} = p * C_{\text{Komplexgebundenes Ca}}$$

$$\Rightarrow C_{\text{freies Citrat}} * C_{iCa} = p * (1{,}2\,\text{mmol/l} - C_{iCa})$$

$$\Rightarrow C_{\text{freies Citrat}} = p * (1{,}2\,\text{mmol/l} - C_{iCa}) / C_{iCa}$$

$$\Rightarrow C_{\text{cit-blut bei Hkt}=0\%} = p * (1{,}2\,\text{mmol/l} - C_{iCa}) / C_{iCa} + v * (1{,}2\,\text{mmol/l} - C_{iCa})$$

| | |
|---|---|
| $C_{iCa}$ | *Zielwert der Konzentration freier Ca-Ionen im antikoagulierten Blut* |

| | |
|---|---|
| $C_{Komplexgebundenes\ Ca}$ | *Konzentration komplexgebundener Ca-Ionen* |
| $C_{cit\_blut}$ | *Citrat-Konzentration im Blut (gesamt)* |
| $C_{Komplexgebundens\ Citrat}$ | *Konzentration komplexgebundenes Citrat* |
| $C_{freies\ Citrat}$ | *Konzentration des freien Citrats* |
| *p, v* | *empirisch gefundene Faktoren (p=l, v=2.5)* |

Die Anpassung der Citrat-Konzentration im Blut an den Patientenhämatokrit (Hkt) erfolgt dabei durch Anwendung folgender Formel:

$$C_{\text{cit-blut bei Hkt[\%]}} = C_{\text{cit-blut bei Hkt=0\%}} * (100\% - \text{Hkt}[\%])/100\%$$

Zur besseren Übersicht sind die den jeweiligen Hämatokritwerten (0%, 25%, 50%) zugehörigen Kennlinien im Diagramm gekennzeichnet und beschriftet. Aus den zuvor abgeleiteten Gleichungen können natürlich beliebige Kennlinien $C_{cit-blut}$ in Abhängigkeit von der iCa Zielkonzentration $G_{iCa}$ und dem Hkt berechnet werden.

Ausgehend vom gewünschten Zielwert und dem initialen Hämatokritwert wird aus der abgebildeten Kurvenschar unter Berücksichtigung der Höhe des Blutflusses die notwendige Citratzufuhrrate $Q_{cit}$ ermittelt. Dies wird anhand des folgenden Berechnungsbeispiels veranschaulicht:

Der initiale Hämatokrit Hkt beträgt 50% und der Zielwert der Ca-Ionenkonzentration nach Citrat-Zugabe soll 0,2 mmol/l sein. Die für eine effektive Antikoagulation notwendige Citratkonzentration im Blut $C_{cit\_blut}$ ist gemäß dem Diagramm in Fig. 3 daher ~ 4,5 mmol/l Unter der Annahme, dass der Blutfluss $Q_B$ = 250 ml/min und die Citratkonzentration in der Infusionslösung $C_{cit}$ = 500 mmol/l beträgt lässt sich die Citratzufuhrrate $Q_{cit}$ aus dem Masseerhaltungssatz $Q_{cit}$ * $C_{cit}$ = $Q_B$ * $C_{cit\_blut}$ berechnen.

$$Q_{cit} = (Q_B * C_{cit\_blut}) / C_{cit}$$

$$= (250\ ml/min * 4{,}5\ mmol/l) / 500\ mmol/l = 2{,}25\ ml/min = 135\ ml/h$$

Die Citratzufuhrrate $Q_{cit}$ wird - zurückkommend auf Fig. 2 - vor Beginn der Behandlung durch Einstellung der Pumpendrehzahl der Citratpumpe 218 eingestellt und nur an Änderungen des Blutflusses $Q_B$ angepasst. Ändert sich der Blutfluss $Q_B$ nicht, bleibt die Citratzufuhrrate $Q_{cit}$ während der gesamten Behandlung gleich. Um auf Änderungen im Blutfluss $Q_B$ zu reagieren, ist die Citratzufuhrrate $Q_{cit}$ über einen konstanten Faktor mit dem Blutfluss $Q_B$ verbunden. Ein Stoppen der Blutpumpe 209 und folglich des Blutflusses wird von der Steuerung 206 erkannt, von dieser an die Steuerung 225 kommuniziert, welche ein Anhalten der Citratpumpe 218 veranlasst. Dadurch wird im Sinne der Patientensicherheit verhindert, dass bei Anhalten der Blutpumpe 209 weiter Citrat in den extrakorporalen Blutkreislauf 202 gepumpt wird. Ebenso wird von der Steuerungseinheit 225 bei einer Änderung der Citratzufuhrrate eine Änderung der Substitutionsmediumzufuhrrate veranlasst bzw. bei Stoppen der Blutpumpe 209 oder der Citratpumpe 218 erfolgt ein Stoppen der Substitutionsmediumpumpe 222.

In der beschriebenen Ausführung wird die Citratzufuhrrate $Q_{cit}$ nicht in Abhängigkeit der vom Ca-Ionen-Sensor 224 gemessenen Ca-Ionenkonzentration eingestellt.

Dies schließt natürlich nicht aus, dass erfindungsgemäß die vom Ca-Ionen-Sensor 224 gemessene Ca-Ionenkonzentration auch eine Regelgröße für die Citratzufuhrrate sein kann.

Die initiale Substitutionsmediumszufuhrrate $Q_{Ca/Mg}$ wird ausgehend von der Citratzufuhrrate $Q_{cit}$ und der Citratclearance des Dialysefilters 205a berechnet und in Abhängigkeit der vom Ca-Ionen-Sensor 224 gemessenen Ca-Ionenkonzentration laut vorheriger Vorschrift geregelt.

Die Substitutionsmediumszufuhrrate $Q_{Ca/Mg}$ wird mittels der Substitutionsmediumspumpe 222 in Abhängigkeit der vom Ca-Ionen-Sensor 224 gemessenen Ca-Ionenkonzentration des Blutes geregelt. Die vom Ca-Ionen-Sensor 224 gemessene Ca-Ionenkonzentration stellt dabei den Istwert dar und wird mit einem Sollwertbereich (z.B. 1,1 - 1,3 mmol/l) verglichen.

- Liegt die Ca-Ionenkonzentration des Blutes im Bereich von 1,1-1,3 mmol/l, wird die Substitutionsmediumszufuhrrate $Q_{Ca/Mg}$ belassen.
- Liegt die Ca-Ionenkonzentration unter 1,1 mmol/l, so wird die Substitutionsmediumszufuhrrate $Q_{Ca/Mg}$ um 10% erhöht.
- Liegt die Ca-Ionenkonzentration über 1,3 mmol/l, so wird die Substitutionsmediumszufuhrrate $Q_{Ca/Mg}$ um 10% erniedrigt.

- Liegt die Ca-Ionenkonzentration unter 1,0 mmol/l oder über 1,4 mmol/l, so wird eine Warnung ausgegeben und der Bediener aufgefordert, einen Arzt zu Rate zu ziehen.

Die Citratpumpe 218 ist über die Steuerung 225 mit der Substitutionsmediumspumpe 222 über einen laut obenstehender Vorschrift berechneten Faktor verbunden. Wird der Blutfluss angehalten, wird über den Steuerungsalgorithmus automatisch die Citratzufuhr und infolge dessen auch die Zufuhr des Substitutionsmediums gestoppt. Dieser Steuerungsalgorithmus ist für die Aufrechterhaltung der Patientensicherheit von hoher Wichtigkeit und verhindert bei einem Ausfall der Blutpumpe 209 eine Akkumulation von Citrat bzw. Ca- und Mg-Ionen im extrakorporalen Blutkreislauf 202.

Die vom Ca-Ionen-Sensor 224 gemessene Ca-Ionenkonzentration spiegelt den aktuellen intrakorporalen Ca-Ionen-Spiegel und somit den physiologischen Zustand des Patienten wieder. Dadurch kann ein Trend in Richtung eines unphysiologischen Zustandes erkannt werden. So kann bei Über- oder Unterschreiten eines Grenzwertbereiches zusätzlich zur Regelung der Substitutionsmediumszufuhrrate $Q_{Ca/Mg}$ ein Alarm ausgelöst werden.

Weiters wird die Citratclearance bei der Substitutionsmediumszufuhrrate berücksichtigt. Die Citrat-Clearance ist ein Merkmal des verwendeten Dialysefilters und ist abhängig vom Blut- und Dialysatfluss. Änderungen der Citrat-Clearance bewirken eine Änderung der Substitutionsmediumszufuhrrate. Dabei wird zu Beginn der Behandlung der Filtertyp vom Bediener in der Steuerung 225 eingegeben. Änderungen im Citrat- und Dialysatfluss werden von der Blutpumpe 209 bzw. der Dialysatpumpe 207a an die Steuerung 206 und von dieser an die Steuerung 225 kommuniziert, welche wiederum eine Anpassung der Substitutionsmediumszufuhr durch Ansteuern der Substitutionsmediumspumpe 222 veranlasst.

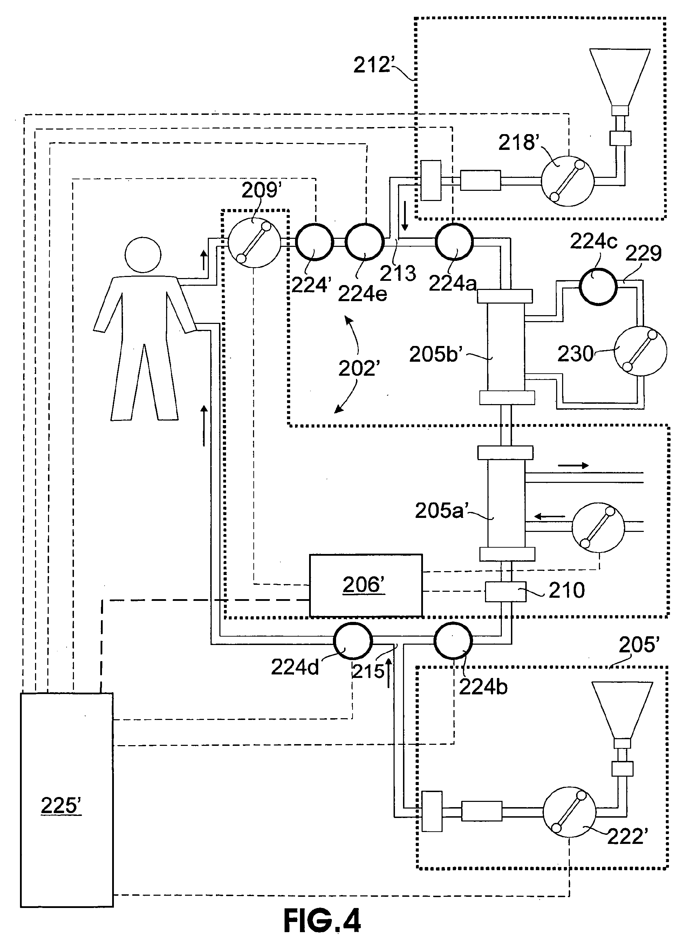

Fig. 4 zeigt eine weitere Ausführung der Erfindung mit Erweiterungen gegenüber der in Fig. 2 dargestellten Ausführungsform. Die in Fig. 4 gezeigte Vorrichtung entspricht jener der Fig. 2, sofern im Folgenden nicht anders angegeben. Die dargestellten Erweiterungen können einzeln oder in Kombination zur Anwendung kommen.

So kann beispielsweise ein zusätzliches Mittel zur Erfassung der Ionenkonzentration - ein Ca-Ionen-Sensor 224a - im extrakorporalen Blutkreislauf 202' zwischen der Citratzufuhrstelle 213 und der Dialyseeinheit 205' angeordnet sein. Die Dialyseeinheit 205' setzt sich aus der Blutpumpe 209', dem Dialysefilter 205a' und der Steuerung 206' zusammen. Der Ca-Ionen-Sensor 224a misst somit die Ca-Ionenkonzentration des antikoagulierten Blutes vor Eintritt in die Dialyseeinheit 205'. Der an dieser Position erhaltene Messwert müsste bei aufrechter effektiver Antikoagulation einen Wert in Übereinstimmung mit dem oben definierten vorgebbaren Zielwert und/ oder Zielwertbereich haben.

Außerdem kann ein zusätzliches Mittel zur Erfassung der Ionenkonzentration - ein Ca-Ionen-Sensor 224b - zwischen der Dialyseeinheit 205' und der Substitutionsmedium-Zufuhrstelle 215 angeordnet sein. Um Störungen der Messung durch etwaig vorhandene Luftblasen zu vermeiden, ist es besser, den Ca-Ionen-Sensor 224b stromab der Luftfalle mit Luftblasendetektor 210 anzuordnen. Der Ca-Ionen-Sensor 224b ermittelt die Ca-Ionenkonzentration nach der Dialyse und ermöglicht somit die exakte Dosierung/Steuerung des Substitutionsmediums.

Beispielsweise ist das zusätzliche Blutreinigungselement 205b ein Hämofilter 205b' mit einem geschlossenen Plasmakreislauf 229 und einer Filtratpumpe 230. In Plasmakreisläufen kann das Plasma beispielsweise mittels eines Adsorbers oder mittels MDS (Microsphere Detoxification System) gereinigt werden. Wenn der Plasmakreislauf 229 geschlossen ist und das Plasma gut durchmischt wird, kann ein zusätzliches Mittel zur Erfassung der Ionenkonzentration - ein Ca-Ionen-Sensor 224c - im Plasmakreislauf 229 angeordnet sein. Aufgrund der guten Durchmischung - das Plasma zirkuliert des öfteren durch den Plasmakreislauf 229 - ist die Ca-Ionenkonzentration im gesamten Plasmakreislauf 229 annähernd gleich und folglich kann der Ca-Ionen-Sensor 224c überall im Plasmakreislauf 229 positioniert werden. Bei Blutreinigungssystemen mit einem Plasmakreislauf 229, bei welchen eine Messung der Ca-Ionenkonzentration zwischen der Citratzufuhrstelle 213 und dem Dialysefilter 205a' vorgesehen ist, kann diese mittels des Ca-Ionen-Sensors 224c zusätzlich oder statt einer Messung mit einem Ca-Ionen-Sensor 224a erfolgen.

Die mittels der Ca-Ionen-Sensoren 224a und 224c erhaltenen Messwerte werden der Steuerung 225' zugeführt und wirken regelnd auf die Citratzufuhrrate $Q_{cit}$, indem die Citratpumpe 218' der Citrat-Dosiereinrichtung 212' von der Steuerung 225' angesteuert wird, wobei die erhaltenen Messwerte die Istwerte darstellen und mit einem Sollwert und/oder Sollwertbereich verglichen werden:

- Liegt die Ca-Ionenkonzentration in einem gewünschten Konzentrationsbereich, in welchem eine effektive Antikoagulation und eine Unterbindung der Komplementaktivierung gewährleistet ist (z.B. 0,2 mmol/l), wird die Citratzufuhrrate $Q_{cit}$ belassen.
- Liegt die Ca-Ionenkonzentration über dem gewünschten Konzentrationsbereich, in welchem eine effektive Antikoagulation gewährleistet ist (z.B. $\geq$ 0,2 mmol/l), so wird die Citratzufuhrrate $Q_{cit}$ der aktuellen Ca-Ionenkonzentration entsprechend erhöht.
- Liegt die Ca-Ionenkonzentration unter dem gewünschten Konzentrationsbereich, so wird die Citratzufuhrrate $Q_{cit}$ der aktuellen Ca-Ionenkonzentration entsprechend reduziert.

Die Sensoren 224a und 224c messen dabei den Istwert und steuern die Citratzufuhr so, dass der Istwert dem voreingestellten Zielwert entspricht. Die Steuerung 225' hat zu überprüfen ob die Citratzufuhrrate innerhalb eines definierten Bereiches liegt um eine unerwünschte Überdosierung zu vermeiden.

Aus Sicherheitsgründen kann eine Änderung der Citratzufuhrrate $Q_{cit}$ wie oben bereits beschrieben eine Änderung der Substitutionsmediumszufuhrrate $Q_{Ca/Mg}$ veranlassen, indem die Subsitutionsmediumspumpe 222' von der Steuerung 225' angesteuert wird. Eine Erhöhung der Citratzufuhrrate $Q_{cit}$ bewirkt eine Erhöhung der Substitutionsmediumszufuhrrate $Q_{Ca/Mg}$ und eine Erniedrigung der Citratzufuhrrate $Q_{cit}$ bewirkt eine Erniedrigung der Substitutionsmediumszufuhrrate $Q_{Ca/Mg}$.

Gemäß einer weiteren in Fig. 4 dargestellten Erweiterung, kann ein zusätzliches Mittel zur Erfassung der Ionenkonzentration - ein Ca-Ionen-Sensor 224d - stromab der Substitutionsmediumszufuhrstelle 215 angeordnet sein. Die Messwerte des Ca-Ionen-Sensors 224d werden in einer weiteren Ausführung der Citrat-Antikoagulation für die Dosierung/Steuerung des Substitutionsmediums verwendet. An dieser Position im extrakorporalen Blutkreislauf 202' soll das Blut wieder physiologische Ca- und Mg-Ionenwerte aufweisen. Die mittels des Ca-Ionen-Sensors 224d erhaltenen Messwerte werden der Steuerung 225' zugeführt und eine Regelung der Substitutionsmediumszufuhrrate $Q_{Ca/Mg}$ entsprechend dem oben beschriebenen Ca-Ionen-Sensor 224 (Fig. 2) durchgeführt.

Große Variationsmöglichkeiten bietet auch die Anordnung der Sensoren. Besonders wichtig für die Umsetzung der Erfindung ist eine möglichst engmaschige Messung einer Ionenkonzentration stromauf der Citratzufuhrstelle 213, welche gemäß der Ausführungsform in Fig. 2 der Ca-Ionen-Sensor 224 ist. Bei der Verwendung eines Ca-Ionen-Sensors, der in regelmäßigen Abständen kalibriert werden muss, kann ein zweiter wesensgleicher Ca-Ionen-Sensor 224e, stromauf der Citratzufuhrstelle 213 angeordnet sein (Fig. 4). Die Ca-Ionenkonzentration wird von den beiden Ca-Ionen-Sensoren 224', 224e alternierend gemessen, wobei einer der Sensoren kalibriert wird, während der andere ein Messsignal generiert. Der zusätzliche Ca-Ionen-Sensor 224e kann auch dazu eingerichtet sein, die Funktion des Ca-Ionen-Sensors 224' zu kontrollieren und bei einer Fehlfunktion einen Alarm auszulösen, z.B. wenn die Messwerte zu stark voneinander abweichen. Der Ca-Ionen-Sensor 224e kann entweder ohne oder mit Unterbrechungen in Betrieb sein. Selbstverständlich kann der Ca-Ionen-Sensor 224e auch für eine andere Ionenart sensitiv sein. Beispielsweise kann aufgrund einer einen Grenzwert überschreitenden Änderung des Verhältnisses der Kalzium- und Magnesiumionen, wobei der Ca-Ionen-Sensor 224' die Ca-Ionenkonzentration und der Ca-Ionen-Sensor 224e die Mg-Ionenkonzentration erfasst, ein Alarm ausgelöst werden.

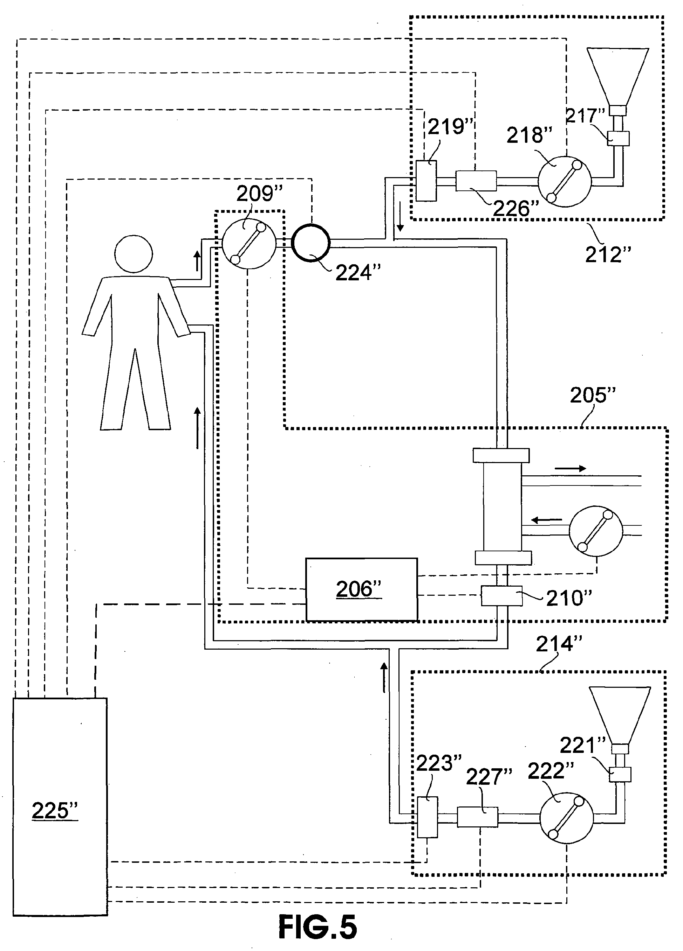

Fig. 5 zeigt eine dritte Ausführungsform, die bei Fehlfunktionen einen Alarm auslösen kann. Die in Fig. 5 gezeigte Vorrichtung entspricht jener der Fig. 2, sofern im Folgenden nicht anders angegeben. Um die Sicherheit für den Patienten möglichst hoch zu halten ist erfindungsgemäß auch vorgesehen, dass die einzelnen Module - die Citrat-Dosiereinrichtung 212", die Substitutionsmedium-Dosiereinrichtung 214" und das extrakorporale Blutreinigungssystem (= die Dialyseeinheit 205") - so miteinander verbunden sind, dass ein Alarm, welcher von einer der Dosiereinrichtungen 212", 214" bzw. der Dialyseeinheit 205" ausgelöst wird, von den jeweils anderen Modulen erkannt wird und entsprechend darauf reagiert wird. Die Kommunikation zwischen den einzelnen Modulen erfolgt über die Steuerungen 206", 225". Der Alarm wird optisch und akustisch angezeigt. Ein Alarm kann beispielsweise durch die folgenden Fehlfunktionen ausgelöst werden:

- Der Ausfall einer der Pumpen 209", 218", 222" löst einen Alarm aus, und bewirkt mittels der Steuerungen 206" bzw. 225" ein Anhalten der restlichen Pumpen.
- Das Abklemmen eines Schlauches in den Dosiervorrichtungen 212", 214" wird durch die Drucksensoren 226", 227" erkannt, welche das Signal an die Steuerung 225" weiterleiten und ein Anhalten der Pumpen 218", 222" bewirken. Über die Steuerung 225" wird das Signal an die Steuerung 206" kommuniziert, welche ein Abschalten der Blutpumpe 209" bewirkt.
- Ist die Citratlösung oder das Substitutionsmedium verbraucht, wird von den Tropfendetektoren 217", 221" ein Alarm ausgelöst, welcher von der Steuerung 225" erkannt wird und ein Abschalten der Pumpen 218", 222" bewirkt. Über die Steuerung 225" wird das Signal an die Steuerung 206" kommuniziert, welche ein Abschalten der Blutpumpe 209" bewirkt.
- Ähnlich den Tropfendetektoren 217", 991", kann von den Luftblasendetektoren 210", 219", 223" ein Alarm ausgelöst werden, welcher von der Steuerung 225" bzw. der Steuerung 206' erkannt und an die jeweils anderen Module weiterkommuniziert wird.
- Weiters kann ein Alarm durch ein Signal des Ca-Ionen-Sensors 224" ausgelöst werden, wenn - wie oben beschrieben - die Ca-Ionenkonzentration einen Sollwertbereich
- unter- oder überschreitet. Dadurch wird ein Trend in Richtung eines unphysiologischen Zustands signalisiert.
- Ein Alarm kann weiters von allen anderen zusätzlich in Fig. 4 gezeigten Ca-Ionen-Sensoren 224', 224e, 224a, 224b, 224c, 224d ausgelöst werden.

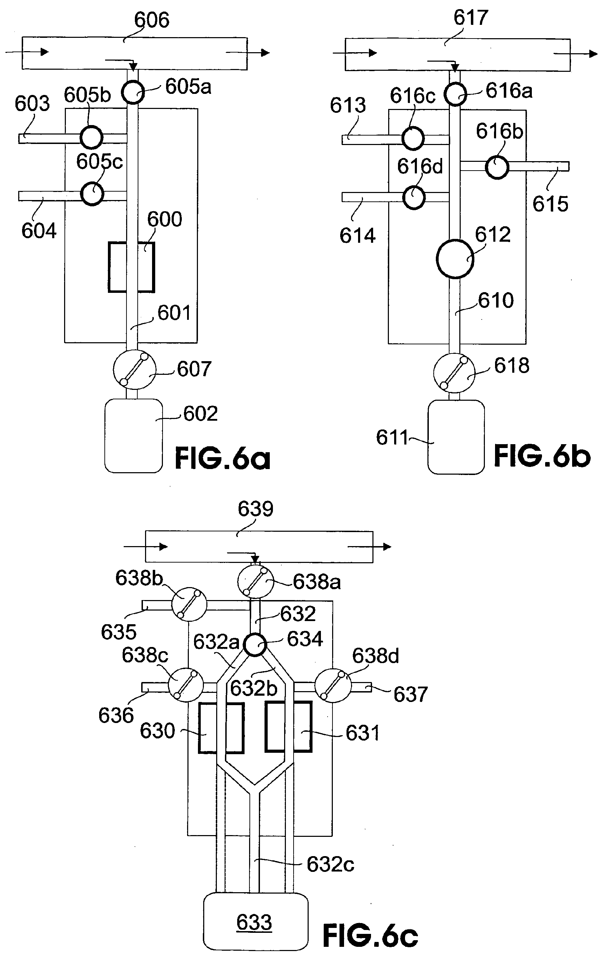

Fig. 6a-e zeigen verschiedene Ausführungsbeispiele für Ca-Ionen-Sensoren. Um den hohen Anforderungen an die Sterilität gerecht zu werden, ist es besonders empfehlenswert, wenn diese Sensoren Einwegartikel sind, die vor Beginn der Behandlung an den oben genannten Positionen (siehe Fig. 4) im extrakorporalen Blutkreislauf oder Plasmakreislauf angeordnet und nach der Behandlung entsorgt werden. Selbstverständlich können auch sterilisierbare Sensoren verwendet werden.

Fig. 6a zeigt einen Ca-Ionen-Sensor mit einer Sensoroberfläche 600, der in einer Bypass-Leitung 601 integriert ist. Eine geringe Menge Blut wird dabei vom extrakorporalen Blutkreislauf 606 in die Bypass-Leitung 601 abgezweigt und auf die Sensoroberfläche 600 geleitet. Die Abzweigung der Blutprobe erfolgt durch eine Pumpe 607. Auf der Sensoroberfläche 600 sind Ca-sensitive Moleküle - beispielsweise Ca-sensitive Fluorophore - immobilisiert, die ein der Ca-Ionenkonzentration entsprechendes Signal (Fluoreszenzsignal) generieren. Eine Änderung der Calciumkonzentration führt bei den ionensenitiven Fluorophoren enweder zu einer Änderung der Fluoreszenzintensität oder zu einer Änderung der Fluoreszenzwellenlänge. Nach der Messung wird die Blutprobe nicht mehr in den extrakorporalen Blutkreislauf 606 rückgeführt, sondern wird in einem Abfallbehältnis 602 gesammelt und anschließend verworfen. Ein Ventil 605a verhindert einen Rückfluss der Blutprobe in den extrakorporalen Blutkreislauf 606. Wird dieses Prinzip bei dem in Fig. 2 beschriebenen Ca-Ionen-Sensor 224, der stromauf der Citratzufuhrstelle 213 angeordnet ist, angewendet, ist eine Antikoagulation aufgrund der niedrigen Blutflussraten in der Bypass-Leitung 601 notwendig. Dies kann durch eine Einspritzung von Antikoagulans über eine Antikoagulans-Leitung 603 in die Bypass-Leitung 601 erfolgen. Der Sensor kann mittels Zugabe einer Kalibrierlösung, welche über eine Kalibrierlösungsleitung 604 in die Bypass-Leitung 601 eingebracht wird, kalibriert werden. In der Antikoagulans-Leitung 603 und der Kalibrierlösungsleitung 604 sind Ventile 605b, 605c angeordnet.

Fig. 6b zeigt ein weiteres Ausgestaltungsbeispiel eines Ca-Ionen-Sensors, wobei ebenfalls eine geringe Menge Blut aus dem extrakorporalen Blutkreislauf 617 über eine Pumpe 618 in eine Bypass-Leitung 610 abgezweigt wird und die Blutprobe nach der Messung in einem Abfallbehältnis 611 gesammelt und verworfen wird. Im Gegensatz zu der in Fig. 6a gezeigten Ausgestaltung sind die Ca-sensitiven Moleküle - z.B. Ca-sensitive Fluorophore - nicht auf einer Sensoroberfläche immobilisiert. Die Ca-sensitiven Moleküle werden mit einer mit Antikoagulans antikoagulierten Blutprobe vermischt und das Signal über ein Detektionsfenster 612 erfasst. Das Antikoagulans wird über eine Antikoagulans-Leitung 613 und die Ca-sensitiven Fluorophore über eine Fluorophor-Leitung 614 der in der Bypass-Leitung 610 befindlichen Blutprobe zugegeben. Die Kalibrierlösung wird über eine Kalibrierlösungsleitung 615 in die Bypass-Leitung 610 eingebracht. Die Vorrichtungselemente 616a, 616b, 616c, 616d können entweder Ventile oder Pumpen sein.

In Fig. 6c ist ein weiteres Ausführungsbeispiel eines Ca-Ionen-Sensors gezeigt, welcher eine erste Sensoroberfläche 630 und eine zweite Sensoroberfläche 631 aufweist. Auf den Sensoroberflächen 630, 631 sind Ca-sensitive Moleküle - beispielsweise Ca-sensitive Fluorophore - immobilisiert, die ein der Ca-Ionenkonzentration entsprechendes Signal (Fluoreszenzsignal) generieren. Mit einem derart ausgestalteten Sensor kann eine redundante bzw. alternierende Messung durchgeführt werden. Bei dieser Ausführung wird eine geringe Menge Blut aus dem extrakorporalen Blutkreislauf 639 in eine Bypass-Leitung 632 abgezweigt. Die Bypass-Leitung 632 teilt sich in zwei weitere Bypass-Leitungen 632a und 632b. Die Bypass-Leitung an 632a und 632b werden später wieder zu einer Bypass-Leitung 632c vereinigt, welche in einem Abfallbehältnis 633 mündet. In einer anderen Variante können die Bypass-Leitungen 632a, 632b auch direkt in das Abfallbehältnis 633 münden (gestrichelte Verlängerungen). Das Blut gelangt aus dem extrakorporalen Blutkreislauf 639 mittels einer ersten Pumpe 638a in die Bypass-Leitung 632 und kann weiters entweder in beide Bypass-Leitungen 632a und 632b oder über ein Ventil 634 entweder in die Bypass-Leitung 632a oder in die Bypass-Leitung 632b geleitet werden. Von dort gelangt das Blut zu der jeweiligen Sensoroberfläche 631, 630. Nach der Messung wird die Blutprobe in das Abfallbehältnis 633 geleitet und verworfen. Antikoagulans wird über eine Antikoagulans-Leitung 635 mittels einer zweiten Pumpe 638b in die Bypass-Leitung 632 eingebracht. Die Kalibrierlösung wird über zwei Kalibrierlösungsleitungen 636, 637 mittels zwei weiterer Pumpen 638c, 638d in die jeweiligen Bypass-Leitungen 632a, 632b eingebracht. Diese Anordnung hat den Vorteil, dass eine redundante oder alternierende Messung mit nur einer vom extrakorporalen Blutkreislauf 202 abzweigenden Leitung möglich ist. Ist das Ventil 634 für beide Bypass-Leitungen 632a, 632b offen fließt die Blutprobe zur gleichen Zeit über beide Sensoroberflächen 630, 631. Das Ventil 634 kann auch dazu eingerichtet sein, dass die Messung alternierend durchgeführt werden kann, wobei die Blutprobe abwechselnd nur in eine der Bypass-Leitungen 632a, 632b und somit zur jeweiligen Sensoroberfläche 630, 631 gelangen kann. Die jeweils andere Bypass-Leitung 632a, 632b bleibt verschlossen und die jeweilige Sensoroberfläche 630, 631 kann mit Kalibrierlösung versetzt werden.

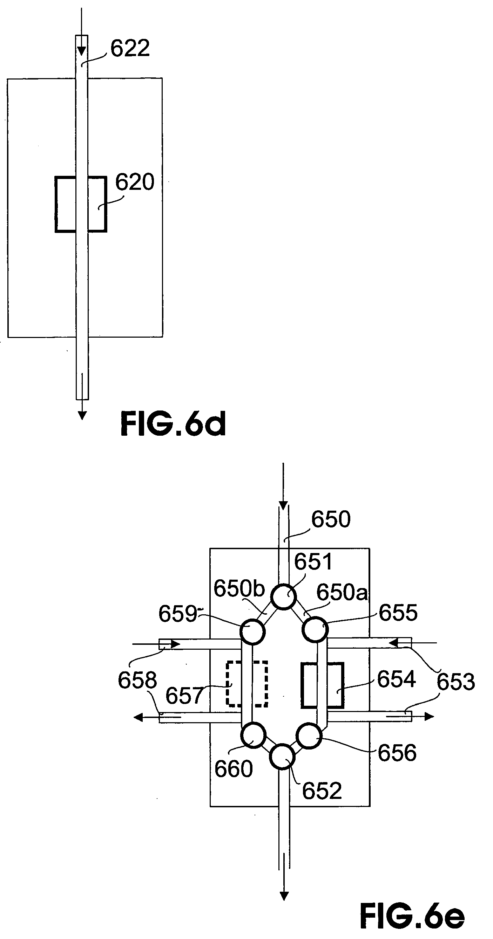

Ein weiteres Ausführungsbeispiel eines Ca-Ionen-Sensors ist in Fig. 6d dargestellt. Hier ist der Sensor, welcher eine Sensoroberfläche 620 mit immobilisierten Ca-sensitiven Molekülen - z.B. Ca-sensitive Fluorophore - aufweist, an der Innenwand des extrakorporalen Blutkreislaufes 622 angeordnet. Der Vorteil dieses Prinzips gegenüber den Ausführungsprinzipien in Fig. 6a, Fig. 6b und Fig. 6c ist die direkte Messung im extrakorporalen Blutkreislauf 622. Es besteht daher keine Notwendigkeit für eine Bypass-Leitung. Das Blut muss nach der Messung nicht entsorgt werden, sondern fließt beständig an der Innenwand des extrakorporalen Blutkreislaufs befindlichen Sensoroberfläche 620 vorbei. Bei dieser Ausführungsform ergeben sich hohe Anforderungen an die Sterilität und Biokompatibilität und der Sensor sollte so geschaffen sein, dass keine Kalibration während der Behandlungsdauer notwendig ist. Wie oben bereits erwähnt, könnte auch der aus der WO 2006/029293 bekannte Sensor mit einer Ca-sensitiven Sensorfläche versehen werden und gemäß diesem Sensorprinzip zur Anwendung kommen.

Die Fig. 6e zeigt ein weiteres Ausführungsbeispiel eines rekalibrierbaren blutseitigen Ca-Ionen-Sensors. Die extrakorporale Blutkreislauf 650 teilt sich in zwei Leitungen 650a, 650b, die sich später wieder zu einer Leitung vereinigen. Das Blut kann dabei - gesteuert über die Ventile 651, 652 - entweder nur über eine der Leitungen 650a, 650b oder durch beide Leitungen fließen. In der Leitung 650a ist eine Sensoroberfläche 654 angeordnet, die gemäß jener in Fig. 6c beschriebenen Sensoroberfläche 620 ausgeführt ist. Wird die Sensoroberfläche 654 kalibriert, fließt das Blut nur über die Leitung 650b. Die Kalibrierlösung wird über eine Kalibrierlösungsleitung 653 zur Sensoroberfläche 654 hin- und von dort wieder abgeleitet. Zwei weitere Ventile 655, 656, die bei diesem Vorgang geschlossen sind, sorgen dafür, dass die Kalibrierlösung nur im Bereich der Sensoroberfläche in den extrakorporalen Blutkreislauf gelangt. Eine alternierende Messung ist möglich, wenn auch in der Leitung 650b eine Sensoroberfläche 657 angeordnet ist. Zur Kalibrierung der Sensoroberfläche 657 sind ebenfalls eine Kalibrierungslösungsleitung 658 und zwei Ventile 659, 660 vorgesehen. Während das Blut über die eine Sensoroberfläche fließt, kann die andere Sensoroberfläche kalibriert werden. Bei Sensoroberflächen, die keiner Rekalibrierung bedürfen, kann mit beiden Sensoroberflächen 654, 657 zur gleichen Zeit gemessen werden.

Selbstverständlich sind die in Fig.6a-e dargestellten Ausgestaltungsbeispiele ebenso für die Ca-Ionen-Sensoren wie sie in Fig. 4 dargestellt sind anwendbar.

Die oben beschriebenen Verwirklichungen der Erfindung sind nur Beispiele unter Vielen und sind folglich nicht als einschränkend zu betrachten.

## Patentansprüche

1. Vorrichtung zur Citrat-antikoagulierten extrakorporalen Blutreinigung, aufweisend:

ein extrakorporales Blutreinigungssystem mit einem extrakorporalen Blutkreislauf (202,202'), welcher eine Dialyseeinheit (205,205',205"), einen Blutzulauf (203) zur Dialyseeinheit (205,205',205") für von einem Patienten (201) entnommenes Blut und einen Blutablauf (204) von der Dialyseeinheit (205,205',205") für in den Patienten (201) zurückzuführendes Blut aufweist,
eine gesteuerte Citrat-Dosiereinrichtung (212) zur Zuführung von Citrat an einer Citratzufuhrstelle (213) stromauf der Dialyseeinheit (205),
eine gesteuerte Substitutionsmedium-Dosiereinrichtung (214) zur Zuführung eines Substitutionsmediums an einer Substitutionsmedium-Zufuhrstelle (215) stromab der Dialyseeinheit (205),
zumindest ein Ionenkonzentrationsmessmittel (224,224',224e,224") zur Messung zweiwertiger Kationen, und
eine Steuerung (225,225',225"), welche mit dem zumindest einen Ionenkonzentrationsmessmittel (224, 224',224e,224") sowie der Citrat-Dosiereinrichtung (212) und der Substitutionsmedium-Dosiereinrichtung (214) verbunden ist, wobei die Steuerung (225,225',225") dazu eingerichtet ist, die Dosierung des Substitutionsmediums in Abhängigkeit eines Vergleichs zwischen einem Sollwertbereich und der mittels des Ionenkonzentrationsmessmittels (224,224',224e,224") gemessenen Ionenkonzentration zu regeln,
**dadurch gekennzeichnet, dass**
das Ionenkonzentrationsmessmittel (224,224',224e,224") zur laufenden Generierung von Messwerten eingerichtet und stromauf der Citratzufuhrstelle (213) angeordnet ist, und die Steuerung (225,225',225") dazu eingerichtet ist, eine Regelung der Dosierung des Citrats und des Substitutionsmediums unter Berücksichtigung eines in der Steuerung (225,225',225") stromab der Citratzufuhrstelle (213) vorgebbaren Zielwerts oder Zielwertbereichs laufend durchzuführen, wobei der stromab der Citratzufuhrstelle (213) vorgebbare Zielwert oder Zielwertbereich jene Kalziumionenkonzentration ist, bei welcher eine Blutgerinnung in einem der Dialyseeinheit (205, 205', 205") zugeordneten Filter (205a, 205a') effektiv verhindert wird, und wobei das Ionenkonzentrationsmessmittel (224, 224', 224e, 224") in den extrakorporalen Blutkreislauf (202, 202') eingesetzt ist.

2. Vorrichtung nach Anspruch 1, **dadurch gekennzeichnet, dass** das Ionenkonzentrationsmessmittel (224,224',224e,224") Erdalkalüonen misst, wobei die Erdalkaliionen gewählt sind aus einer Gruppe bestehend aus Kalziumionen und Magnesiumionen.

3. Vorrichtung nach einem der Ansprüche 1 oder 2, **dadurch gekennzeichnet, dass** das Ionenkonzentrationsmessmittel (224,224',224e,224") ein ionensensitiver Sensor, insbesondere ein optischer ionensensitiver Sensor, ist.

4. Vorrichtung nach einem der Ansprüche 1 bis 3, **gekennzeichnet durch** ein zusätzliches Ionenkonzentrationsmessmittel (224a) stromab der Citratzufuhrstelle (213) und stromauf der Dialyseeinheit (205').

5. Vorrichtung nach einem der Ansprüche 1 bis 4, **gekennzeichnet durch** ein zusätzliches Ionenkonzentrationsmessmittel (224b) stromab der Dialyseeinheit (205') und stromauf der Substitutionsmedium-Zufuhrstelle (215).

6. Vorrichtung nach einem der Ansprüche 1 bis 5, **gekennzeichnet durch** ein zusätzliches Ionenkonzentrationsmessmittel (224d) stromab der Substitutionsmedium-Zufuhrstelle (215).

7. Vorrichtung nach einem der Ansprüche 1 bis 6, **gekennzeichnet durch** einen Plasmakreislauf (229) mit einem zusätzlichen Ionenkonzentrationsmessmittel (224c).

8. Vorrichtung nach einem der Ansprüche 1 bis 7, **dadurch gekennzeichnet, dass** die Dosiereinrichtungen (212",214") für Citrat und für das Substitutionsmedium und das extrakorporale Blutreinigungssystem über die Steuerung (225") mittels Signalverbindungen miteinander verbunden sind, wobei die Steuerung (225") dazu eingerichtet ist, ihr zugeführte Signale des extrakorporalen Blutreinigungssystems in ihre Regelung einzubeziehen.

9. Vorrichtung nach einem der Ansprüche 1 bis 8, **dadurch gekennzeichnet, dass** die Dosiereinrichtungen (212",214") für Citrat und für das Substitutionsmedium und das extrakorporale Blutreinigungssystem über die Steuerung (225") mittels Signalverbindungen miteinander verbunden sind, wobei die Steuerung (225") dazu eingerichtet ist, ihr zugeführte Signale der Dosiereinrichtungen (212", 214") in ihre Regelung einzubeziehen.

10. Vorrichtung nach einem der Ansprüche 1 bis 9, **gekennzeichnet durch** eine Filtrationseinrichtung (205, 205b) des extrakorporalen Blutreinigungssystems, wobei die Dosiereinrichtungen (212,214) für Citrat und für das Substitutionsmedium und das extrakorporale Blutreinigungssystem über die Steuerung (225) mittels Signalverbindungen miteinander verbunden sind, und wobei die Steuerung (225) dazu eingerichtet ist, bei einer Änderung der Citratzufuhrrate und/oder der Substitutionsmediumszufuhrrate eine proportionale Änderung der Ultrafiltrationsmenge der Filtrationseinrichtung (200, 205b) zu veranlassen oder einem Bediener vorzuschlagen.

11. Vorrichtung nach einem der Ansprüche 1 bis 10, **dadurch gekennzeichnet, dass** der Zielwert die Kalziumionenkonzentration des antikoagulierten Blutes repräsentiert und in einem Bereich von 0,15 - 0,5 mmol/l wählbar ist.

12. Vorrichtung nach Anspruch 11, **dadurch gekennzeichnet, dass** der Zielwert in einem Bereich zwischen 0,2 - 0,4 mmol/l wählbar ist.

## Claims

1. A device for the citrate anti-coagulated extracorporeal blood purification comprising:

   an extracorporeal blood purification system comprising an extracorporeal blood circuit (202, 202'), which comprises a dialysis unit (205, 205', 205''), a blood inflow (203) to the dialysis unit (205, 205', 205'') for blood drawn from a patient (201) and a blood discharge (204) from the dialysis unit (205, 205', 205'') for blood to be returned into the patient (201),
   a controlled citrate metering device (212) for supplying citrate at a citrate supply point (213) upstream of the dialysis unit (205),
   a controlled substitution medium metering device (214) for supplying a substitution medium at a substitution medium supply point (215) downstream from the dialysis unit (205), at least one ion concentration measuring means (224, 224', 224e, 224'') for measuring bivalent cations and
   a controller (225, 225', 225''), which is connected to the at least one ion concentration measuring means (224, 224', 224e, 224'') as well as to the citrate metering device (212) and to the substitution medium metering device (214), wherein the controller (225, 225', 225'') is adapted to regulate the metering of the substitution medium as a function of a comparison between a setpoint value range and the ion concentration measured by means of the ion concentration measuring means (224, 224', 224e, 224''),
   **characterized in that**
   the ion concentration measuring means (224, 224', 224e, 224'') is adapted for the continuous generation of measuring values and is arranged upstream of the citrate supply point (213) and the controller (225, 225', 225'') is adapted to continuously carry out a regulation of the metering of the citrate and of the substitution medium in consideration of a target value or target value range, which can be predetermined in the controller (225, 225', 225'') downstream from the citrate supply point (213),

wherein the target value or target value range, which can be predetermined downstream from the citrate supply point (213), represents the calcium ion concentration in the case of which a blood clotting in a filter (205a, 205a') assigned to the dialysis unit (205, 205', 205'') is effectively prevented, and wherein the ion concentration measuring means (224, 224', 224e, 224'')is introduced into the extracorporeal blood circuit.

2. The device according to claim 1, **characterized in that** the ion concentration measuring means (224, 224', 224e, 224'') measures alkaline earth ions, wherein the alkaline earth ions are selected from group consisting of calcium ions and magnesium ions.

3. The device according to one of claims 1 or 2, **characterized in that** the ion concentration measuring means (224, 224', 224e, 224'') is an ion-sensitive sensor, in particular an optical ion-sensitive sensor.

4. The device according to one of claims 1 to 3, **characterized by** an additional ion concentration measuring means (224a) downstream from the citrate supply point (213) and upstream of the dialysis unit (205').

5. The device according to one of claims 1 to 4, **characterized by** an additional ion concentration measuring means (224b) downstream from the dialysis unit (205') and upstream of the substitution medium supply point (215).

6. The device according to one of claims 1 to 5, **characterized by** an additional ion concentration measuring means (224d) downstream from the substitution medium supply point (215).

7. The device according to one of claims 1 to 6, **characterized by** a plasma circuit (229) comprising an additional ion concentration measuring means (224c).

8. The device according to one of claims 1 to 7, **characterized in that** the metering devices (212", 214") for citrate and for the substitution medium and the extracorporeal blood purification system are connected to one another via the controller (225") by means of signal connections, wherein the controller (225") is adapted to include signals from the extracorporeal blood purification system supplied to it into its regulation.

9. The device according to one of claims 1 to 8, **characterized in that** the metering devices (212", 214") for citrate and for the substitution medium and the extracorporeal blood purification system are connected to one another via the controller (225") by means of signal connections, wherein the controller (225") is adapted to include signals from the metering devices (212", 214") supplied to it into its regulation.

10. The device according to one of claims 1 to 9, **characterized by** a filtration device (205, 205b) of the extracorporeal blood purification system, wherein the metering devices (212, 214) for citrate and for the substitution medium and the extracorporeal blood purification system are connected to one another via the controller (225) by means of signal connections, and wherein the controller (225) is adapted to initiate a proportional change of the ultrafiltration quantity of the filtration device (200, 205b) or to propose this to an operator in response to a change of the citrate supply rate and/or of the substitution medium supply rate.

11. The device according to one of claims 1 to 10, **characterized in that** the target value represents the calcium ion concentration of the anti-coagulated blood and can be chosen in a range between 0.15-0.5 mmol/l.

12. The device of claim 11, **characterized in that** the target value can be chosen in a range between 0.2-0.4 mmol/l.

**Revendications**

1. Dispositif de purification extracorporelle de sang soumis à une anticoagulation au citrate, comprenant :

un système de purification extracorporelle de sang comportant un circuit extracorporel de sang (202, 202'), lequel présente une unité de dialyse (205, 205', 205''), une entrée de sang (203) dans l'unité de dialyse (205, 205', 205'') pour du sang prélevé à partir d'un patient (201) et une sortie de sang (204) à partir de l'unité de dialyse (205, 205', 205'') pour du sang retournant dans le patient (201) ;
un dispositif de dosage de citrate (212) commandé pour l'amenée de citrate à un point d'amenée de citrate (213) en amont de l'unité de dialyse (205) ;

un dispositif de dosage de milieu de substitution (214) commandé pour l'amenée d'un milieu de substitution à un point d'amenée de milieu de substitution (215) en aval de l'unité de dialyse (205) ;

au moins un moyen (224, 224', 224e, 224'') de mesure de la concentration en ions pour la mesure de cations divalents ;

et

un dispositif de commande (225, 225', 225''), lequel est relié avec le ou les moyens (224, 224', 224e, 224'') de mesure de concentration en ions ainsi qu'au dispositif de dosage de citrate (212) et au dispositif de dosage de milieu de substitution (214), le dispositif de commande (225, 225', 225'') étant adapté pour réguler le dosage du milieu de substitution en fonction d'une comparaison entre une plage de valeurs de consigne et la concentration en ions mesurée à l'aide du moyen (224, 224', 224e, 224'') de mesure de concentration en ions, **caractérisé par le fait que**

le moyen (224, 224', 224e, 224'') de mesure de la concentration en ions est adapté pour générer en continu des valeurs de mesure et est disposé en amont du point d'amenée de citrate (213), et le dispositif de commande (225, 225', 225'') est adapté pour effectuer en continu une régulation du dosage du citrate et du milieu de substitution en considération d'une valeur cible prédéterminée ou d'un domaine de valeurs cibles prédéterminé dans le dispositif de commande (225, 225', 225'') en aval du point d'amenée de citrate (213), la valeur cible prédéterminée ou le domaine de valeurs cibles prédéterminé en aval du point d'amenée de citrate (213) étant la concentration en ions calcium à laquelle une coagulation du sang est efficacement empêchée dans un filtre (205a, 205a') associé à l'unité de dialyse (205, 205', 205''), et le moyen (224, 224', 224e, 224'') de mesure de la concentration en ions étant placé dans le circuit extracorporel de sang (202, 202').

2. Dispositif selon la revendication 1, **caractérisé par le fait que** le moyen (224, 224', 224e, 224'') de mesure de la concentration en ions mesure les ions alcalino-terreux, les ions alcalino-terreux étant choisis dans un groupe consistant en ions calcium et ions magnésium.

3. Dispositif selon l'une des revendications 1 ou 2, **caractérisé par le fait que** le moyen (224, 224', 224e, 224'') de mesure de la concentration en ions est un capteur sensible aux ions, en particulier un capteur optique sensible aux ions.

4. Dispositif selon l'une des revendications 1 à 3, **caractérisé par** un moyen supplémentaire (224a) de mesure de la concentration en ions en aval du point d'amenée de citrate (213) et en amont de l'unité de dialyse (205').

5. Dispositif selon l'une des revendications 1 à 4, **caractérisé par** un moyen supplémentaire (224b) de mesure de la concentration en ions en aval de l'unité de dialyse (205') et en amont du point d'amenée de milieu de substitution (215).

6. Dispositif selon l'une des revendications 1 à 5, **caractérisé par** un moyen supplémentaire (224d) de mesure de la concentration en ions en aval du point d'amenée de milieu de substitution (215).

7. Dispositif selon l'une des revendications 1 à 6, **caractérisé par** un circuit de plasma (229) comportant un moyen supplémentaire (224c) de mesure de la concentration en ions.

8. Dispositif selon l'une des revendications 1 à 7, **caractérisé par le fait que** les dispositifs de dosage (212'', 214'') pour le citrate et pour le milieu de substitution et le système de purification extracorporelle de sang sont reliés entre eux par l'intermédiaire du dispositif de commande (225'') au moyen de liaisons par signaux, le dispositif de commande (225'') étant adapté pour intégrer dans sa régulation les signaux du système de purification extracorporelle de sang qui lui sont amenés.

9. Dispositif selon l'une des revendications 1 à 8, **caractérisé par le fait que** les dispositifs de dosage (212'', 214'') pour le citrate et pour le milieu de substitution et le système de purification extracorporelle de sang sont reliés entre eux par l'intermédiaire du dispositif de commande (225'') au moyen de liaisons par signaux, le dispositif de commande (225'') étant adapté pour intégrer dans sa régulation les signaux des dispositifs de dosage (212'', 214'') qui lui sont amenés.

10. Dispositif selon l'une des revendications 1 à 9, **caractérisé par** un dispositif de filtration (205, 205b) du système de purification extracorporelle de sang, les dispositifs de dosage (212, 214) pour le citrate et pour le milieu de substitution et le système de purification extracorporelle de sang étant reliés entre eux par l'intermédiaire du dispositif de commande (225) au moyen de liaisons par signaux, et le dispositif de commande (225) étant adapté, lors d'une modification du débit d'amenée de citrate et/ou du débit d'amenée de milieu de substitution, pour amener une modification proportionnelle de la quantité d'ultrafiltration du dispositif de filtration (200, 205b) ou pour la proposer à un utilisateur.

11. Dispositif selon l'une des revendications 1 à 10, **caractérisé par le fait que** la valeur cible représente la concentration en ions calcium du sang anticoagulé et est apte à être choisie dans une plage de 0,15 - 0,5 mmol/l.

12. Dispositif selon la revendication 11, **caractérisé par le fait que** la valeur cible est apte à être choisie dans une plage entre 0,2 - 0,4 mmol/l.

**FIG.1(Stand der Technik)**

**FIG.2**

FIG.3

**FIG.4**

**FIG.5**

FIG.6a

FIG.6b

FIG.6c

**FIG.6d**

**FIG.6e**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0776223 A
- US 5855782 A
- WO 9106326 A
- US 2007066928 A1
- US 2007066928 A
- US 2004133145 A1
- US 20070007184 A1
- EP 1175917 A
- DE 10114283 C2
- WO 2006029293 A
- US 4344438 A
- DE 10114283

**In der Beschreibung aufgeführte Nicht-Patentliteratur**

- **HETZEL GR ; SUCKER C.** *Nephrol Dial Transplant,* 2005, vol. 20 (10), 2036-2042
- **M. FRANCHINI.** *Thrombosis Journal,* 2005, vol. 3 (14
- **JANSSEN MJ et al.** *Nephrol Dial Transplant,* 1993, vol. 8 (11), 1228-1233
- **PINNICK et al.** *N Engl J Med,* 1983, vol. 308, 258-261
- **PALSSON R ; NILES JL.** *Kidney Int,* 1999, vol. 55, 1991-1997
- **BÖHLER et al.** *J Am Soc of Nephrol.,* 1996, vol. 7 (2), 234-241
- **KUTSOGIANNIS et al.** *Am J Kidney Dis,* 2000, vol. 35, 802-811
- **TOLWANI et al.** *Kidney International,* 2001, vol. 60, 370-374
- **SWARTZ et al.** *Clinical Nephrology,* 2004, vol. 61 (2), 134-143
- **SCHREFL A ; KELLNER KH ; HARTMANN J ; STROBL W ; FALKENHAGEN D.** A novel monitor for Anticoagulation with citrate. *Int. J. Artif. Organs,* 2001, vol. 24 (8), 15